# EUROPEAN PATENT APPLICATION

(11) **EP 4 693 311 A1**
(43) Date of publication of application: **11.02.2026**
(21) Application number: 24778580.1
(22) Date of filing: 19.01.2024
(51) Int. Cl.: G16C 20/80, G06F 16/9038, G16C 20/00

(54) **INFORMATION PROCESSING DEVICE, INFORMATION PROCESSING METHOD, AND PROGRAM**

(30) Priority: 31.03.2023 JP 2023059425
(71) Applicant: FUJIFILM Corporation, Tokyo 106-8620 (JP)
(72) Inventor: TATESHITA, Masakazu, Ashigarakami-gun, Kanagawa 258-8577 (JP); HIKIDA, Yasushi, Ashigarakami-gun, Kanagawa 258-8577 (JP); MURAKAMI, Ryoichi, Ashigarakami-gun, Kanagawa 258-8577 (JP); SUGIYAMA, Satoshi, Ashigarakami-gun, Kanagawa 258-8577 (JP)
(74) Representative: HGF
(86) International application number: PCT/JP2024/001439
(87) International publication number: WO 2024/202434

(57) **Abstract**

An information processing apparatus includes a processor, in which the processor is configured to generate a display image in which marks indicating a compound group including a target compound, which is a compound to be subjected to an toxicity evaluation, and a plurality of reference compounds, which are compounds to be compared with the target compound, are mapped onto a chemical space in which a plurality of feature amounts related to a molecular structure of a compound are set as coordinate axes, in which the marks are displayed such that a result of the toxicity evaluation for each of the compounds is distinguishable, and execute control of outputting the generated display image.

## Description

### BACKGROUND OF THE INVENTION

### 1. Field of the Invention

The technology of the present disclosure relates to an information processing apparatus, an information processing method, and a program.

### 2. Description of the Related Art

In recent years, in the field of handling chemical substances, a toxicity evaluation using a computer-based in silico method has become common. The toxicity evaluation of the chemical substance requires, in addition to simply obtaining the toxicity evaluation, various examinations from the viewpoint of causes of toxicity manifestation or a tendency of a compound that is likely to manifest toxicity.

WO2020/100310A discloses a compound design support method comprising: a selection step of selecting one or a plurality of compounds; a characteristic determination step of determining characteristics of each of the selected compounds using a characteristic determination learning network trained with a condition for determining the characteristics; a space definition step of defining a chemical space having, as an axis, a parameter in a spatialization layer, which is one of interlayers of the characteristic determination learning network; and a plotting step of plotting each of the selected compounds on coordinates in the chemical space based on a parameter value in the spatialization layer.

JP2003-527649A discloses a computer-based method for identifying one or more compounds having one or more similar properties, the method including: using a computer to identify characteristics of a target compound and to execute similarity binding to identify one or more database compounds having characteristics similar to the characteristics of the target compound.

JP2022-545252A discloses a computer-implemented method including: receiving an input of a biological target or a ligand; receiving an input of characteristics of a generated compound; receiving at least one generative model trained with a reference compound; generating a structure of the generated compound using each generative model such that the generated compound is designed to interact with the biological target and/or correlate with a structural feature of the ligand; prioritizing the structure of the generated compound of each generative model based on at least one reward criterion; processing the prioritized chemical structures of the generated compounds via a Sammon mapping protocol to obtain a hit structure; and providing the chemical structure of the hit structure.

### SUMMARY OF THE INVENTION

In the technologies disclosed in WO2020/100310A, JP2003-527649A, and JP2022-545252A, although the evaluation of a molecular structure of a compound using a chemical space is taken into consideration, no consideration has been given to making it easier to understand a relationship between a position of a compound in a chemical space and a toxicity evaluation result. Therefore, there is room for improvement in analyzing the toxicity evaluation result of the compound and supporting a user in evaluating toxicity of the compound.

One embodiment according to the technology of the present disclosure provides an information processing apparatus, an information processing method, and a program that enable a user to understand a relationship between a toxicity evaluation result and a position of a compound in a chemical space and that are capable of supporting the user in evaluating toxicity of the compound.

A first aspect according to the technology of the present disclosure is an information processing apparatus comprising: a processor, in which the processor is configured to generate a display image in which marks indicating a compound group including a target compound, which is a compound to be subjected to an toxicity evaluation, and a plurality of reference compounds, which are compounds to be compared with the target compound, are mapped onto a chemical space in which a plurality of feature amounts related to a molecular structure of a compound are set as coordinate axes, in which the marks are displayed such that a result of the toxicity evaluation for each of the compounds is distinguishable, and execute control of outputting the generated display image.

A second aspect according to the technology of the present disclosure is the information processing apparatus according to the first aspect, in which the plurality of feature amounts are feature amounts obtained by performing dimensionality reduction processing on the compound group.

A third aspect according to the technology of the present disclosure is the information processing apparatus according to the first aspect, in which the plurality of feature amounts are indexes related to a molar mass and lipophilicity of the compound.

A fourth aspect according to the technology of the present disclosure is the information processing apparatus according to the first aspect, in which, in the display image, a display region for displaying details of results of the toxicity evaluation for the reference compounds is provided separately from the chemical space, and in the display region, the details of the results of the toxicity evaluation for the reference compounds are displayed in ascending order of a distance from the target compound in the chemical space.

A fifth aspect according to the technology of the present disclosure is the information processing apparatus according to the first aspect, in which, in the display image, a display range of the chemical space is changeable to a predetermined range centered on the mark indicating the target compound.

A sixth aspect according to the technology of the present disclosure is the information processing apparatus according to the first aspect, in which, in the display image, in a case where the mark corresponding to the reference compound in the chemical space is selected, details of a result of the toxicity evaluation for the selected reference compound are displayed.

A seventh aspect according to the technology of the present disclosure is the information processing apparatus according to the first aspect, in which a display aspect of the mark in the chemical space is changed according to a result of the toxicity evaluation, so that the result of the toxicity evaluation is made distinguishable in the display image.

An eighth aspect according to the technology of the present disclosure is the information processing apparatus according to the first aspect, in which a display aspect of the mark indicating the target compound in the chemical space is distinguishable from a display aspect of the mark indicating the reference compound.

A ninth aspect according to the technology of the present disclosure is the information processing apparatus according to the first aspect, in which the chemical space is a two-dimensional chemical space in which two coordinate axes are set.

A tenth aspect according to the technology of the present disclosure is the information processing apparatus according to the first aspect, in which the display image is displayed by transitioning from a list image in which results of the toxicity evaluation for a plurality of the compounds are displayed in a list, in a case where at least one target compound is selected from the list image.

An eleventh aspect according to the technology of the present disclosure is the information processing apparatus according to the tenth aspect, in which, in a case where a user designates a range in the chemical space in the display image, the compounds included in the range are filtered out from the plurality of compounds in the list image.

A twelfth aspect according to the technology of the present disclosure is an information processing method comprising: generating a display image in which marks indicating a compound group including a target compound, which is a compound to be subjected to an toxicity evaluation, and a plurality of reference compounds, which are compounds to be compared with the target compound, are mapped onto a chemical space in which a plurality of feature amounts related to a molecular structure of a compound are set as coordinate axes, in which the marks are displayed such that a result of the toxicity evaluation for each of the compounds is distinguishable; and executing control of outputting the generated display image.

A thirteenth aspect according to the technology of the present disclosure is a program for causing a computer to execute a process comprising: generating a display image in which marks indicating a compound group including a target compound, which is a compound to be subjected to an toxicity evaluation, and a plurality of reference compounds, which are compounds to be compared with the target compound, are mapped onto a chemical space in which a plurality of feature amounts related to a molecular structure of a compound are set as coordinate axes, in which the marks are displayed such that a result of the toxicity evaluation for each of the compounds is distinguishable; and executing control of outputting the generated display image.

The technology of the present disclosure provides an information processing apparatus, an information processing method, and a program that enable a user to understand a relationship between a toxicity evaluation result and a position of a compound in a chemical space and that are capable of supporting the user in evaluating toxicity of the compound.

### BRIEF DESCRIPTION OF THE DRAWINGS

FIG. 1 is a conceptual diagram showing a schematic configuration example of an information processing system.
FIG. 2 is a block diagram showing an example of a hardware configuration of an electrical system of a server.
FIG. 3 is a conceptual diagram showing an example of a usage aspect of the information processing system.
FIG. 4 is a functional block diagram showing an example of main functions of a processor of the server.
FIG. 5 is a conceptual diagram showing an example of processing contents of a toxicity evaluation unit.
FIG. 6 is a conceptual diagram showing an example of processing contents of a feature amount derivation unit and a coordinate setting unit.
FIG. 7 is a conceptual diagram showing an example of processing contents of an image generation unit.
FIG. 8 is a conceptual diagram showing an example of processing contents of the image generation unit.
FIG. 9 is a conceptual diagram showing an example of an aspect in which a part of a display image is displayed on a display device.
FIG. 10 is a conceptual diagram showing an example of the display image.
FIG.11 is a conceptual diagram showing an example of the display image.
FIG. 12 is a conceptual diagram showing an example of a result detail image.
FIG. 13 is a flowchart showing an example of a flow of server control processing.
FIG. 14 is a conceptual diagram showing an example of processing contents of the feature amount derivation unit, the coordinate setting unit, and the image generation unit.
FIG. 15 is a conceptual diagram showing an example of an aspect in which a range is designated in a chemical space image.
FIG. 16 is a block diagram showing an example of a hardware configuration of an electrical system of a client terminal.
FIG. 17 is a functional block diagram showing an example of main functions of a processor of the client terminal.
FIG. 18 is a functional block diagram showing an example of main functions of the processor of the client terminal.

### DESCRIPTION OF THE PREFERRED EMBODIMENTS

An example of embodiments of an information processing apparatus, an information processing method, and a program according to the technology of the present disclosure will be described with reference to the accompanying drawings.

### [First Embodiment]

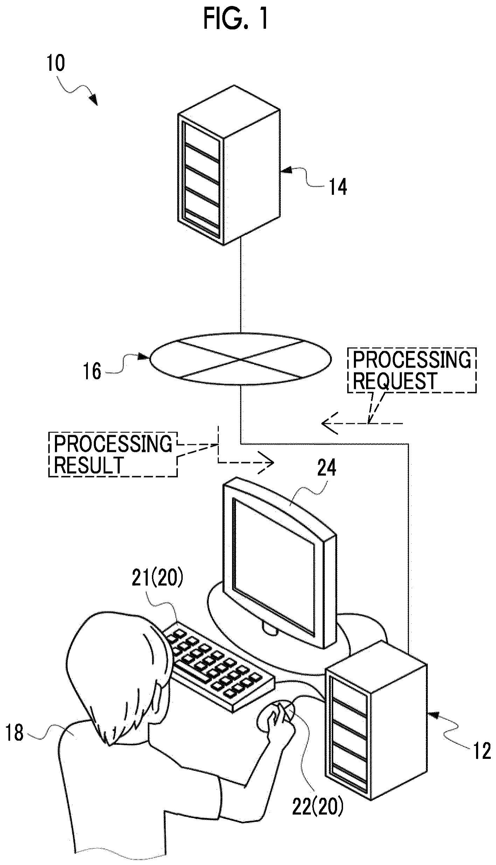

As shown in FIG. 1 as an example, an information processing system 10 comprises a client terminal 12 and a server 14. In the information processing system 10, the client terminal 12 and the server 14 are communicably connected to each other via a network 16.

The information processing system 10 is used, for example, for evaluation of characteristics of a chemical substance (for example, evaluation of the presence or absence of toxicity to a human body). A user 18 (for example, a researcher) can use the information processing system 10 to predict and evaluate experimental results using computer simulations (that is, an evaluation using in silico method). For example, for a newly developed or under-development chemical substance, it is possible to obtain an evaluation result of toxicity without performing a toxicity evaluation test (that is, an in vitro or in vivo toxicity evaluation test) using an actual chemical substance.

The client terminal 12 is a terminal used by the user 18. The server 14 receives a processing request from the client terminal 12 via the network 16 and provides a service corresponding to the request to the client terminal 12 that has made the request via the network 16. For example, in a case where the server 14 receives a request to execute processing related to a characteristic evaluation of a chemical substance as the processing request, the server 14 transmits an evaluation result to the client terminal 12. In the present embodiment, the server 14 is an example of an "information processing apparatus" according to the technology of the present disclosure.

For example, the server 14 is realized by a mainframe, but this is merely an example. For example, the server may be realized by cloud computing, or may be realized by network computing such as fog computing, edge computing, or grid computing. Here, the server 14 is exemplified as an example of a device provided outside the client terminal 12, but this is merely an example, and at least one personal computer or the like may be used instead of the server 14.

The network 16 is configured by, for example, at least one of a wide area network (WAN) or a local area network (LAN). Further, a connection method between the client terminal 12 and the network 16 and a connection method between the server 14 and the network 16 may be a wireless communication method or a wired communication method. The network 16 establishes communication between the client terminal 12 and the server 14, and transmits and receives various types of information between the client terminal 12 and the server 14.

A reception device 20 is connected to the client terminal 12. The reception device 20 receives an instruction from the user 18. The reception device 20 includes a keyboard 21, a mouse 22, and the like. The keyboard 21 and the mouse 22 shown in FIG. 1 are merely an example. As the reception device 20, any one of the keyboard 21 or the mouse 22 may be provided. In addition, as the reception device 20, for example, at least one of a proximity input device that receives a proximity input, a voice input device that receives a voice input, or a gesture input device that receives a gesture input may be applied instead of the keyboard 21 and/or the mouse 22. The proximity input device is, for example, a touch panel or a tablet.

A display device 24 is connected to the client terminal 12. Examples of the display device 24 include an electro-luminescence (EL) display and a liquid crystal display. The display device 24 displays various types of information (for example, image and text) under the control of the client terminal 12.

Here, a personal computer is described as an example of the client terminal 12, but this is merely an example. As the client terminal 12, a mobile terminal, such as a smartphone and a tablet terminal, may be used. Here, although an example in which one client terminal 12 is connected to one server 14 via the network 16 has been described, this is merely an example. It is needless to say that the information processing system 10 may include a plurality of client terminals 12 and a plurality of servers 14.

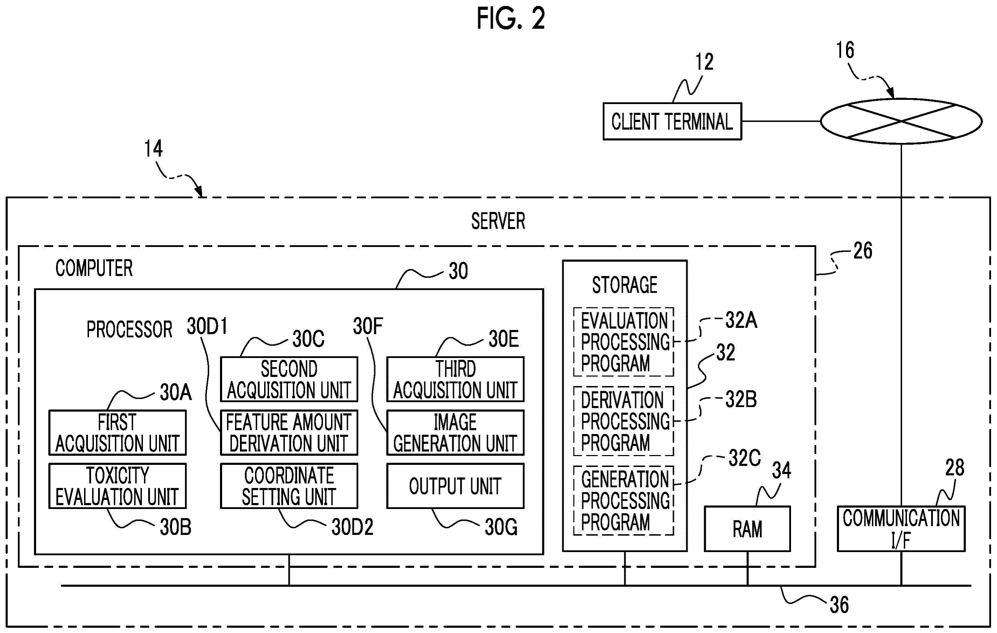

As shown in FIG. 2 as an example, the server 14 comprises a computer 26, a communication interface (I/F) 28, and a bus 36. The computer 26 comprises a processor 30, a storage 32, and a random access memory (RAM) 34. The processor 30, the storage 32, the RAM 34, and the communication I/F 28 are connected to the bus 36. In the present embodiment, the computer 26 is an example of a "computer" according to the technology of the present disclosure, and the processor 30 is an example of a "processor" according to the technology of the present disclosure.

A memory is connected to the processor 30. The memory includes the storage 32 and the RAM 34. The processor 30 includes, for example, a central processing unit (CPU) and a graphics processing unit (GPU). The GPU operates under the control of the CPU, and is responsible for executing processing related to an image.

The storage 32 is a non-volatile storage device that stores various programs, various parameters, and the like. Examples of the storage 32 include a flash memory (for example, an electrically erasable and programmable read only memory (EEPROM) and a solid state drive (SSD)), and/or a hard disk drive (HDD). The flash memory and the HDD are merely examples, and at least one of the flash memory, the HDD, a magnetoresistive memory, or a ferroelectric memory may be used as the storage 32.

The RAM 34 is a memory that transitorily stores information, and is used as a work memory by the processor 30. Examples of the RAM 34 include a dynamic random access memory (DRAM) or a static random access memory (SRAM).

The communication I/F 28 is connected to the network 16. The communication I/F 28 is responsible for transmitting and receiving information to and from an external communication device (for example, the client terminal 12) via the network 16. For example, the communication I/F 28 transmits information in response to a request from the processor 30 to the external communication device via the network 16. In addition, the communication I/F 28 receives the information transmitted from the external communication device, and outputs the received information to the processor 30 via the bus 36.

An evaluation processing program 32A is stored in the storage 32. The evaluation processing program 32A is a program that provides an evaluation simulation of toxicity of a chemical substance. The processor 30 reads out the evaluation processing program 32A from the storage 32 and executes the read-out evaluation processing program 32A on the RAM 34 to perform toxicity evaluation processing. The toxicity evaluation processing is realized by the processor 30 operating as a first acquisition unit 30A and a toxicity evaluation unit 30B.

In addition, a derivation processing program 32B is stored in the storage 32. The derivation processing program 32B is a program for deriving coordinates of a compound in a chemical space. The processor 30 reads out the derivation processing program 32B from the storage 32 and executes the read-out derivation processing program 32B on the RAM 34 to perform coordinate derivation processing. The coordinate derivation processing is realized by the processor 30 operating as a second acquisition unit 30C, a feature amount derivation unit 30D1, and a coordinate setting unit 30D2.

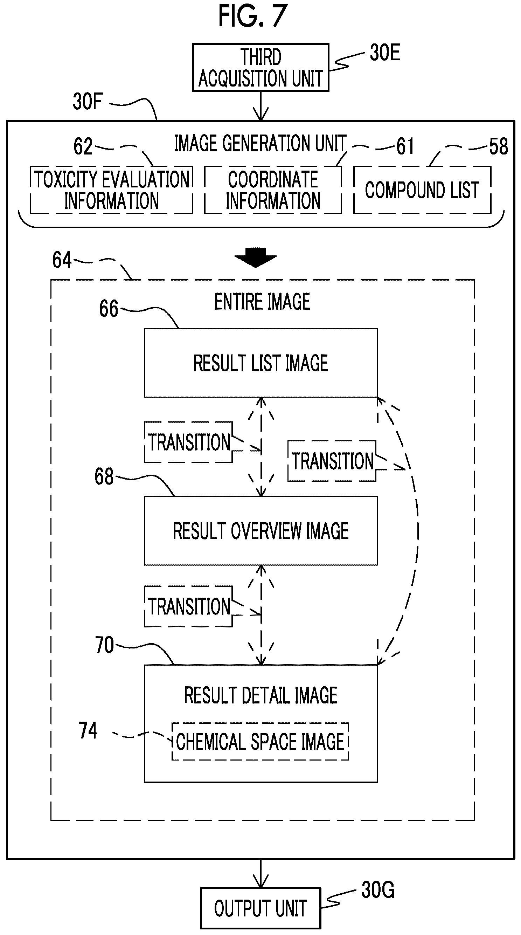

In addition, a generation processing program 32C is stored in the storage 32. The generation processing program 32C is a program that generates an entire image 64 (see FIG. 7 and the like) to be output to the client terminal 12. The processor 30 reads out the generation processing program 32C from the storage 32 and executes the read-out generation processing program 32C on the RAM 34 to perform image generation processing. The image generation processing is realized by the processor 30 operating as a third acquisition unit 30E, an image generation unit 30F, and an output unit 30G. In the present embodiment, the generation processing program 32C is an example of a "program" according to the technology of the present disclosure.

Here, a case where the user 18 uses the information processing system 10 to make an evaluation of toxicity to a human body (hereinafter, also simply referred to as a "toxicity evaluation") for a newly developed chemical substance A will be considered. Here, the toxicity refers to toxicity that the user 18 is interested in as an evaluation item, and examples thereof include mutagenicity, sensitization, and irritation. The mutagenicity refers to a property of causing an irreversible change in genetic information (a base sequence of deoxyribonucleic acid (DNA) or a structure or number of chromosomes) of an organism. The sensitization refers to a property of causing an allergic reaction upon exposure to a chemical substance. The irritation refers to a property in which a chemical substance or the like gives a stimulus (for example, pain or a burning sensation) to the tactile sense or the like.

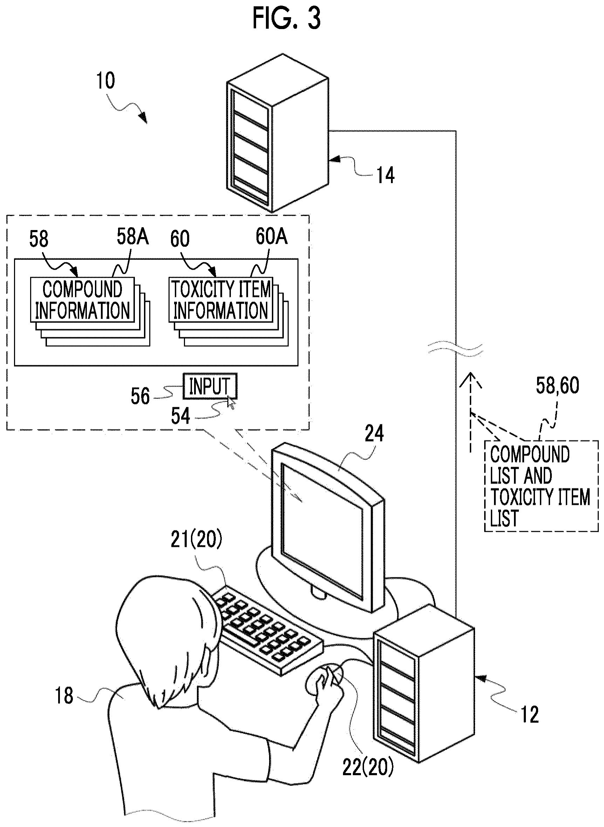

In a case of evaluating the toxicity, as shown in FIG. 3 as an example, the user 18 inputs a compound list 58 to the client terminal 12 via the reception device 20. The compound list 58 is a list of chemical substances that are targets of the toxicity evaluation. Examples of the chemical substance include a low-molecular-weight compound (for example, a compound having a molecular weight of less than 1000), a sugar, a peptide, a protein, and a nucleic acid. The compound list 58 includes a plurality of pieces of compound information 58A. The compound information 58A is information for specifying a chemical structure of the compound. More specifically, the compound information 58A is text information describing a chemical structure such as a structural formula, a compositional formula, and an amino acid sequence, or numerical information obtained by converting such text information into a numerical value. In addition, the compound list 58 may be a list of information indicating a chemical abstract service (CAS) number or a name of a chemical substance. In this case, the client terminal 12 or the server 14 refers to table data or the like in which a correspondence relationship between a CAS number or a name of a chemical substance and a chemical structure is recorded, and reads out the compound information 58A corresponding to the input chemical substance.

In addition, the compound list 58 may be a list of image data showing a chemical structure of a compound as a compound graph (that is, a data structure in which atoms are nodes and bonds are edges). In this case, the client terminal 12 or the server 14 refers to table data or the like in which a correspondence relationship between the compound graph and the compound information 58A is recorded, and reads out the compound information 58A corresponding to the selected compound graph.

In addition, the user 18 inputs a toxicity item list 60, which is a list of toxicity evaluation items, to the client terminal 12 via the reception device 20. Examples of the toxicity evaluation item include an evaluation item based on an AMES test and an evaluation item of skin irritation. The toxicity item list 60 includes a plurality of pieces of toxicity item information 60A. The toxicity item information 60A is information for specifying the toxicity evaluation item.

In the example shown in FIG. 3, a screen for selecting the compound list 58 and the toxicity item list 60 is displayed on a screen of the display device 24, and the user 18 performs a selection operation on the selection screen to select, for example, the compound list 58 and the toxicity item list 60. On the selection screen, for example, the user 18 clicks an input soft key 56 by operating a pointer 54 displayed on the screen of the display device 24 via the mouse 22. As a result, the compound list 58 and the toxicity item list 60 are transmitted from the client terminal 12 to the server 14, and a processing request is made regarding the toxicity evaluation for the compound.

Here, although an example of a form in which the plurality of pieces of compound information 58A are listed and input at once has been described, it is needless to say that each of the plurality of pieces of compound information 58A may be individually input or may be input by being divided into a plurality of lists. In addition, one piece of compound information 58A may be input, and the toxicity of one compound and metabolites may be evaluated.

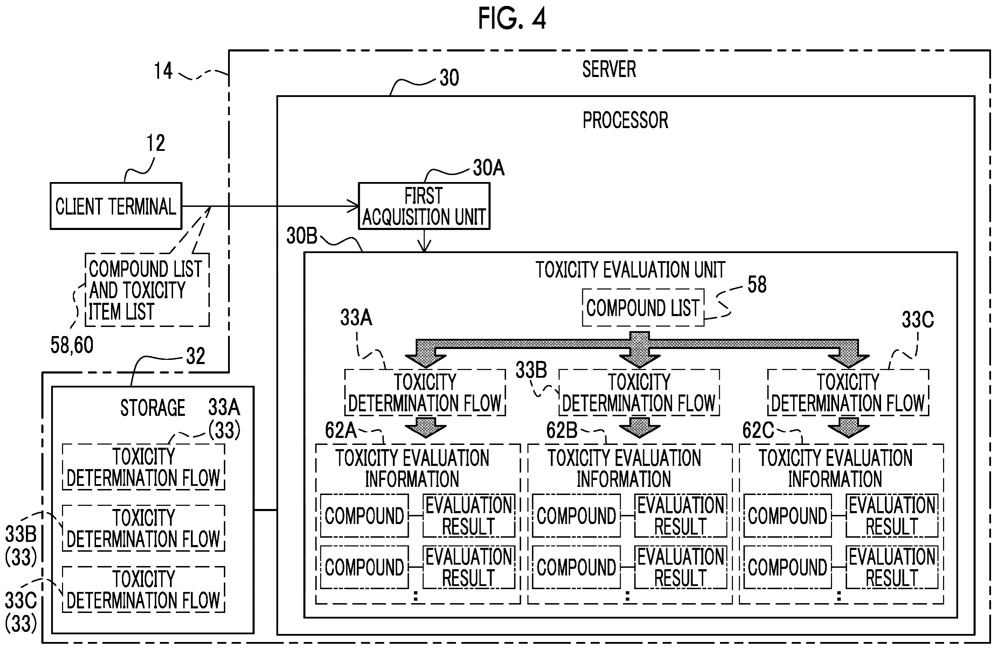

As shown in FIG. 4 as an example, the compound list 58 and the toxicity item list 60 are output from the client terminal 12 to the server 14. In the processor 30 of the server 14, the toxicity evaluation processing is executed. The toxicity evaluation processing is processing of evaluating the toxicity of a compound. In the toxicity evaluation processing, the first acquisition unit 30A outputs the acquired compound list 58 and toxicity item list 60 to the toxicity evaluation unit 30B. The toxicity evaluation unit 30B evaluates the toxicity of a plurality of compounds indicated by the compound information 58A of the compound list 58.

Specifically, the toxicity evaluation unit 30B executes the toxicity evaluation processing according to a toxicity determination flow 33. The toxicity determination flow 33 is described in the evaluation processing program 32A, and is schematically shown in a form of being stored in the storage 32 in FIG. 4. The toxicity determination flow 33 is a determination flow used for evaluating the toxicity. The toxicity determination flow 33 has at least one determination step, and has a route that branches according to a determination result in the determination step. Determination items in each determination step are predetermined. Examples of the determination item include whether or not a compound disappears through metabolism, whether or not a compound permeates a cell membrane, and whether or not a compound corresponds to a structural-alert rule. The structural-alert rule is a regulation related to a chemical structure, and is a rule for specifying a partial structure that may have toxicity of interest to the user. The toxicity determination flow 33 is an example of a "determination flow" according to the technology of the present disclosure.

In addition, the toxicity determination flow 33 is linked to the toxicity evaluation item, and the number of determination steps and the determination content for each determination step are different for each toxicity evaluation item. In the example shown in FIG. 4, toxicity determination flows 33A to 33C corresponding to different evaluation items are displayed in a distinguishable manner as the toxicity determination flow 33.

The toxicity evaluation unit 30B acquires the toxicity item information 60A included in the toxicity item list 60. Then, the toxicity evaluation unit 30B selects the toxicity determination flows 33A to 33C according to the toxicity evaluation items indicated by the toxicity item information 60A.

The toxicity evaluation unit 30B inputs the compound list 58 to each of the toxicity determination flows 33A to 33C. The toxicity evaluation unit 30B executes a determination regarding toxicity evaluation for each determination step on the compound information 58A in each of the toxicity determination flows 33A to 33C. The toxicity evaluation unit 30B outputs toxicity evaluation information 62A to 62C as toxicity evaluation results according to the respective toxicity determination flows 33A to 33C to be executed. Steps ST1 to ST5 described below are examples of a "determination step" according to the technology of the present disclosure.

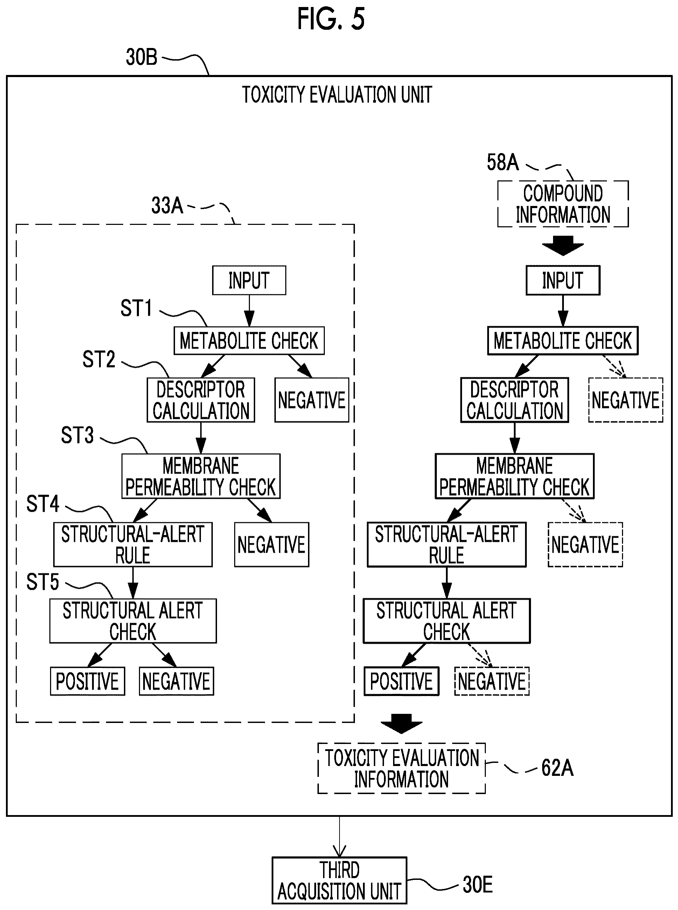

In the following description of the toxicity determination flow 33, the toxicity determination flow 33A will be described as an example. As shown in FIG. 5 as an example, the compound information 58A is input to the toxicity determination flow 33A. In the toxicity determination flow 33A, in step ST1, it is determined whether or not the compound indicated by the compound information 58A is a compound that persists in the body without being metabolically degraded. In a case where the determination in step ST1 is affirmative, the toxicity evaluation for the compound proceeds to step ST2. In a case where the determination in step ST1 is negative, as a result of the toxicity evaluation for the compound, it is determined that the compound is a compound (hereinafter, also simply referred to as a "disappearance compound") that is degraded and disappears in the body, and a negative (that is, non-toxic) evaluation result is output.

Here, in a metabolic reaction, a metabolic enzyme forms a complex with a compound that fits into an active site of the metabolic enzyme. The metabolic enzyme has an action of promoting a metabolic reaction by lowering the energy (that is, the activation energy) required for the compound to cause a chemical reaction. The term "disappearance compound" refers to a compound that, in a case of forming a complex with a metabolic enzyme, has lower activation energy than other metabolites, undergoes a chemical reaction more rapidly than other metabolites, and is converted into the next metabolite in a short period of time. Therefore, the disappearance compound is less likely to react with DNA in a cell, and the necessity of evaluating toxicity (that is, positive or negative evaluation) is low.

In step ST2, descriptor calculation is executed on the compound information 58A. Examples of the descriptor include a molar mass or a LogP (a value obtained by taking the common logarithm of an octanol/water partition coefficient) of the compound. After the process of step ST2 is executed, the toxicity evaluation proceeds to step ST3.

In step ST3, it is determined whether or not the compound can permeate the cell membrane based on a result of the descriptor calculation of the compound executed in step ST2. The numerical value indicated by the descriptor is compared with a threshold value, and a determination is made as to whether or not the compound can permeate the cell membrane based on a comparison result. In a case where the determination in step ST3 is affirmative, the toxicity evaluation proceeds to step ST4. In a case where the determination in step ST3 is negative, a negative (that is, non-toxic) evaluation result is output as the result of the toxicity evaluation. This is because the compound does not permeate the cell membrane and is therefore considered to have little effect on the body.

In step ST4, the structural-alert rule is applied to the compound information 58A. After the process of step ST4 is executed, the toxicity evaluation proceeds to step ST5.

In step ST5, it is determined whether or not a molecular structure indicated by the compound information 58A includes a structural alert. In a case where the determination in step ST5 is negative, a negative (that is, non-toxic) evaluation result is output as the result of the toxicity evaluation. This is because the compound does not have a structural alert and is therefore considered to have a low likelihood of exhibiting toxicity.

In a case where the determination in step ST5 is affirmative, a positive (that is, toxic) evaluation result is output as the result of the toxicity evaluation. In the example shown in FIG. 5, paths and determination steps taken in the toxicity determination flow 33A are shown as the toxicity evaluation for the compound, and an example in which the compound is finally evaluated as positive is shown. In addition, the toxicity evaluation information 62A includes information indicating the result of the toxicity evaluation as well as information indicating a path (hereinafter, also simply referred to as an "evaluation path") leading to the result of the toxicity evaluation in the toxicity determination flow 33A, information indicating an explanation of the determination item, and information indicating details of a determination result for each determination step.

As described above, the toxicity determination flow 33A outputs the toxicity evaluation information 62A according to the input compound information 58A. In the toxicity evaluation information 62, the evaluation result is linked to each compound (see FIG. 4). In this way, the toxicity evaluation processing is executed. The toxicity evaluation unit 30B outputs the toxicity evaluation information 62 to the third acquisition unit 30E.

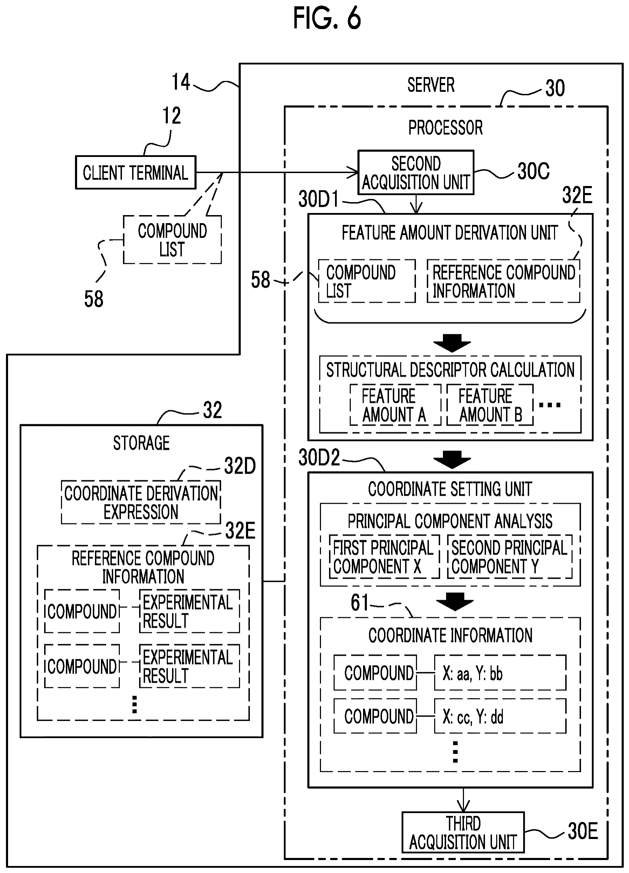

As shown in FIG. 6 as an example, feature amount derivation processing is executed by the processor 30 of the server 14. The feature amount derivation processing is processing of deriving a feature amount related to a molecular structure of a compound. In the feature amount derivation processing, the second acquisition unit 30C outputs the acquired compound list 58 to the feature amount derivation unit 30D1. The feature amount derivation unit 30D1 executes structural descriptor calculation processing of calculating a feature amount indicated by a structural descriptor for a plurality of compounds indicated by the compound list 58. The structural descriptor is a descriptor that describes characteristics of a chemical structure. A type of the structural descriptor is, for example, set in advance by the user 18. As a result of the structural descriptor calculation, a plurality of feature amounts are obtained for each of the plurality of compounds. The feature amount is a value indicating a characteristic related to a chemical structure of a compound.

Then, coordinate setting processing is executed by the processor 30. The coordinate setting processing is processing of setting coordinates of a compound in a chemical space. Here, the chemical space is a space in which feature amounts related to a molecular structure of a compound are set as coordinate axes. By showing the position coordinates corresponding to the compound in the chemical space, it is possible to visualize comparison between characteristics of a certain compound and characteristics of other compounds, or to visualize a group of a plurality of compounds exhibiting similar characteristics.

The coordinate setting unit 30D2 reduces the number of the feature amounts by performing dimensionality reduction processing on a plurality of feature amounts of each compound. In the example shown in FIG. 6, an example is shown in which a first principal component X and a second principal component Y are obtained as the feature amounts by performing principal component analysis as the dimensionality reduction processing. The coordinate setting unit 30D2 acquires a coordinate derivation expression 32D from the storage 32. The coordinate derivation expression 32D is an arithmetic expression used for the principal component analysis, and is an arithmetic expression in which a plurality of feature amounts are independent variables and the first principal component and the second principal component are dependent variables. The first principal component X and the second principal component Y are examples of "feature amounts obtained by performing dimensionality reduction processing" according to the technology of the present disclosure.

Then, the coordinate setting unit 30D2 sets coordinates in the chemical space for the plurality of compounds indicated by the compound list 58. The coordinate setting unit 30D2 generates coordinate information 61 based on the result of the dimensionality reduction processing. The coordinate information 61 is information indicating the position coordinates of the compound in the chemical space. In the example shown in FIG. 6, in the coordinate information 61, the compound is linked to the first principal component X and the second principal component Y.

Here, although an example of a form in which the dimensionality reduction processing is performed using the principal component analysis has been described, this is merely an example. In the dimensionality reduction processing, a known dimensionality reduction method such as t-distributed stochastic neighbor embedding (tSNE) or uniform manifold approximation and projection (UMAP) may be used.

In addition to the compound indicated by the compound list 58 input by the user 18, coordinates in the chemical space are also set for a reference compound. Here, the reference compound is a compound to be compared with the compounds indicated by the compound list 58. The reference compound has an evaluation result of toxicity through an actual experiment. By comparing the toxicity evaluation results of the reference compound and the compound indicated by the compound list 58, it is possible to understand the tendency of toxicity manifestation. The feature amount derivation unit 30D1 acquires reference compound information 32E from the storage 32. Here, the reference compound information 32E is information for specifying a molecular structure of the reference compound and an experimental result of the reference compound. In the example shown in FIG. 6, in the reference compound information 32E, the reference compound and the experimental result are linked to each other. The coordinate setting unit 30D2 also sets coordinates in the chemical space for the reference compound indicated by the reference compound information 32E. As a result, the coordinate information 61 includes the position coordinates of the reference compound in the chemical space. The coordinate setting unit 30D2 outputs the coordinate information 61 to the third acquisition unit 30E.

As shown in FIG. 7 as an example, the third acquisition unit 30E outputs the acquired toxicity evaluation information 62, compound list 58, and coordinate information 61 to the image generation unit 30F. The image generation unit 30F generates an entire image 64 based on the toxicity evaluation information 62, the compound list 58, and the coordinate information 61. The entire image 64 is an image in which the result of the toxicity evaluation can be displayed. The entire image 64 includes a result list image 66, a result overview image 68, and a result detail image 70. The result list image 66 is an image showing a list of compounds together with an evaluation result of each compound. The result overview image 68 is an image showing an overview of evaluation results of toxicity items of a compound.

The result list image 66, the result overview image 68, and the result detail image 70 can transition from one another. For example, in a case where a certain compound is selected in the result list image 66, a transition is made to the result overview image 68 for the compound. Then, in a case where a certain toxicity item is selected in the result overview image 68, a transition is made to the result detail image 70 for the toxicity item. In addition, a transition between the result detail image 70 and the result list image 66 is also possible. The result list image 66 is an example of a "list image" according to the technology of the present disclosure.

In addition, the result detail image 70 is an image showing details of evaluation results of the toxicity items of a compound. The result detail image 70 includes a chemical space image 74. The chemical space image 74 is an image showing a chemical space onto which marks corresponding to compounds are mapped.

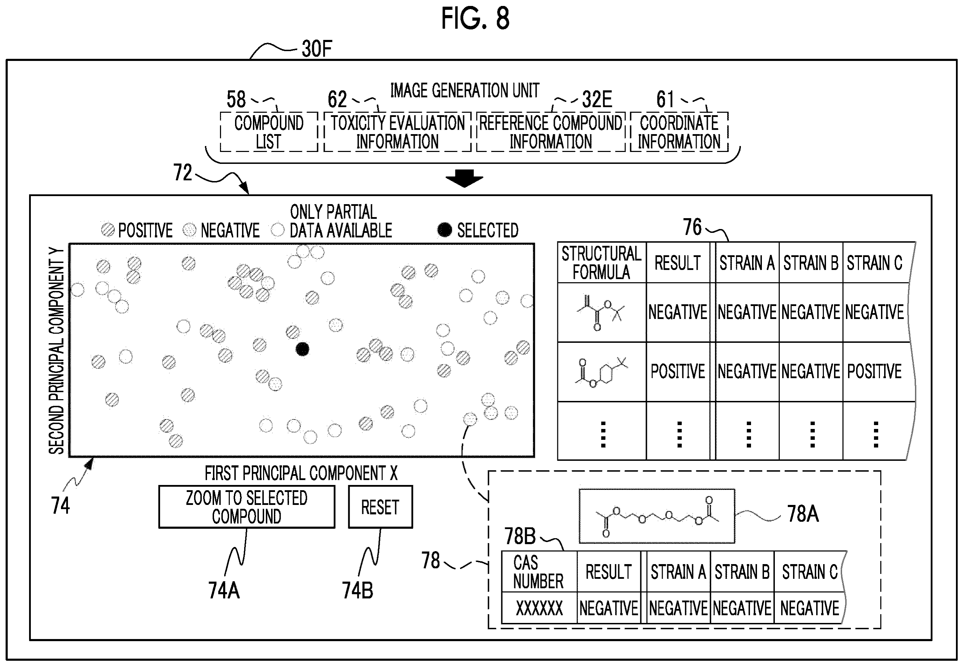

As shown in FIG. 8 as an example, the image generation unit 30F generates an analysis image 72 based on the compound list 58, the toxicity evaluation information 62, the reference compound information 32E, and the coordinate information 61. The analysis image 72 is an image used for analyzing the evaluation result regarding the toxicity item selected in the result detail image 70 for the compound (hereinafter, also simply referred to as a "target compound") shown in the result detail image 70. The analysis image 72 includes the chemical space image 74, a neighboring compound list image 76, and a compound explanation image 78. The analysis image 72 is an example of a "display image" according to the technology of the present disclosure.

In the example shown in FIG. 8, the chemical space image 74 is shown in a left portion of the analysis image 72. The image generation unit 30F generates the chemical space image 74 by mapping marks corresponding to the respective compounds onto the chemical space based on the coordinate information 61. Here, a two-dimensional chemical space in which a horizontal axis represents a first principal component and a vertical axis represents a second principal component is shown. In the chemical space indicated by the chemical space image 74, circular marks corresponding to the target compound and the compounds indicated by the reference compound information 32E are mapped.

In addition, the image generation unit 30F changes a display aspect of the marks mapped onto the chemical space by using the toxicity evaluation result indicated by the toxicity evaluation information 62. That is, the marks are displayed such that the result of the toxicity evaluation for each of the compounds is distinguishable. In the example shown in FIG. 8, a mark of a compound evaluated as "positive" in the toxicity evaluation is indicated as a circle hatched with diagonal lines. In addition, a mark of a compound evaluated as "negative" in the toxicity evaluation is indicated as a circle hatched with dots. In addition, a mark of a compound for which experimental data is available only partially is indicated as an open circle.

In addition, the display aspect of the mark corresponding to the target compound is displayed to be distinguishable from the display aspect of the mark corresponding to the reference compound. In the example shown in FIG. 8, only the mark corresponding to the target compound is indicated by a black circle. As a result, the mark corresponding to the target compound can be distinguished.

In addition, a zoom key 74A and a reset key 74B are displayed below the chemical space image 74. The zoom key 74A is a soft key for changing a predetermined range (for example, a size of 10% with respect to a size of the chemical space image 74 in a case where all the marks are included) centered on the mark corresponding to the target compound. In addition, the reset key 74B is a soft key for returning to the initially displayed range.

The image generation unit 30F generates the neighboring compound list image 76 based on the experimental result indicated by the reference compound information 32E. The neighboring compound list image 76 is an image showing a list of toxicity evaluation results of the compounds shown in the chemical space. On a right side of the chemical space image 74, the neighboring compound list image 76 is shown. In the neighboring compound list image 76, toxicity evaluation results from the past experiment are shown in ascending order of a distance from the target compound in the chemical space. Here, toxicity evaluation results from the AMES test are shown as the neighboring compound list image 76. Items in the columns of the neighboring compound list image 76 are "structural formula", "result", "strain A", "strain B", and "strain C" in order from the left. In each row of the neighboring compound list image 76, contents corresponding to the items of the columns for each compound are described. The column of "result" shows a final toxicity evaluation result based on an experimental result of each strain. In addition, a toxicity evaluation result of a strain used in the AMES test is shown in each of the columns of "strain A", "strain B", and "strain C". The neighboring compound list image 76 is an example of a "display region" according to the technology of the present disclosure.

In addition, the mark displayed in the chemical space image 74 is associated with the compound explanation image 78 for a compound corresponding to the mark. The image generation unit 30F generates the compound explanation image 78 by associating the coordinates of each compound on the chemical space based on the coordinate information 61 with the experimental result indicated by the reference compound information 32E. The compound explanation image 78 is an image showing details of the toxicity evaluation results of the compounds shown in the chemical space.

The compound explanation image 78 includes a structural formula image 78A and an evaluation result table 78B. The structural formula image 78A is an image showing a structural formula of a compound selected in the chemical space. In addition, the evaluation result table 78B is a table showing an evaluation result of the compound selected in the chemical space. Items in the columns of the evaluation result table 78B are "structural CAS number", "result", "strain A", "strain B", and "strain C" in order from the left. In the evaluation result table 78B, contents corresponding to the items of the columns are described. The column of "result" shows a final toxicity evaluation result based on an experimental result of each strain. In addition, an evaluation result of a strain used in the AMES test is shown in each of the columns of "strain A", "strain B", and "strain C".

Although an example of a form in which the evaluation results of the AMES test for each strain are displayed as "positive" or "negative" has been described here, this is merely an example. For example, in a case where the toxicity item is a carcinogenicity test, the evaluation result table 78B may display a specific activity value (that is, an index indicating strength of mutagenicity) for each strain.

In addition, in a case where the toxicity item is a skin sensitization test (Local Lymph Node Assay), a five-stage index (for example, negative, weak, moderate, strong, and extreme) and/or an index value of EC3 may be displayed in the evaluation result table 78B. Further, in a case where the toxicity item is a skin irritation test, a four-stage index (for example, outside category (no irritation), category 3 (mild irritation), category 2 (irritation), and category 1 (corrosiveness)) may be displayed in the evaluation result table 78B.

Although the analysis image 72 shown here is a case where the AMES test is selected as the toxicity item, this is merely an example. The analysis image 72 is generated for each toxic item selected in the result detail image 70.

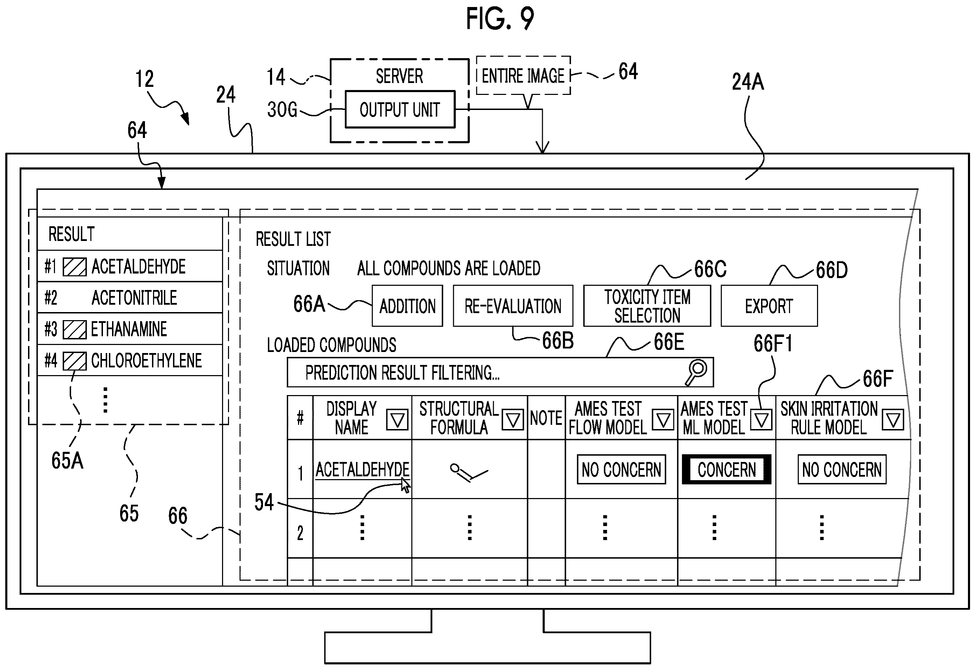

The image generation unit 30F outputs the generated entire image 64 to the output unit 30G. As shown in FIG. 9 as an example, the output unit 30E of the server 14 executes control of outputting the entire image 64 including the analysis image 72 to the client terminal 12. In other words, the server 14 transmits information indicating the entire image 64 including the analysis image 72 to the client terminal 12. On the display device 24 of the client terminal 12, a screen 24A including the entire image 64 is displayed.

A sidebar image 65 is displayed at a left end portion of the entire image 64. The sidebar image 65 shows a list of compounds to be evaluated. In the example shown in FIG. 9, in the sidebar image 65, "acetaldehyde, acetonitrile, ethanamine, and chloroethylene" are shown as the compounds to be evaluated. In addition, a mark 65A indicating that the result of the toxicity evaluation of the compound is "positive" is displayed on a left side of the compound name. In the entire image 64, even in a case where the result list image 66, the result overview image 68, and the result detail image 70 transition from one another, the sidebar image 65 is always displayed. Therefore, it is easy for the user 18 to check the list of evaluation results.

The result list image 66 is displayed on a right side of the sidebar image 65 of the entire image 64.

The result list image 66 comprises an addition key 66A, a re-evaluation key 66B, an item selection key 66C, and an export key 66D. The addition key 66A is a soft key that is operated in a case of adding the compound information 58A indicating the compound to be evaluated. The re-evaluation key 66B is a soft key for re-evaluating the toxicity in a case where the compound information 58A is changed or the toxicity item is changed. The item selection key 66C is a soft key for changing the toxicity item. The export key 66D is a soft key for outputting the evaluation result to the outside.

In addition, a search bar 66E is displayed in the result list image 66. By inputting a text into the search bar 66E, compounds that match the text are filtered out from the compounds to be evaluated. The text that can be input for the search may be, for example, a name of a compound or a character string describing a CAS number or a molecular structure.

In the result list image 66, a result list table 66F showing a list of the evaluation results of the toxicity items for each compound is shown. In the example shown in FIG. 9, items in the columns of the result list table 66F are "#", "display name", "structural formula", "note", "AMES test flow model", "AMES test ML model", and "skin irritation rule model" in order from the left. In each row of the result list table 66F, contents corresponding to the items of the columns are described. In the example shown in FIG. 9, the content regarding acetaldehyde of #1 (for example, the structural formula and the evaluation result of the toxicity) is described.

In the following description, for convenience of description, the entire image 64 will be described using acetaldehyde, which is a known substance, as an example, but it is needless to say that the technology of the present disclosure can be applied to a new substance.

In a first row of the result list table 66F, a filter button 66F1 is provided in each column. Each column can be sorted in ascending or descending order by operating the filter button 66F1. As described above, in the result list table 66F, the compounds displayed in the result list table 66F can be filtered out and/or sorted according to a condition designated by the user 18 by using the search bar 66E and/or the filter button 66F1.

In addition, by selecting a display name in the sidebar image 65 and the result list table 66F, the result list image 66 transitions to the result overview image 68. In the example shown in FIG. 9, the display name "acetaldehyde" in the result list table 66F is selected via the pointer 54, and a transition to the result overview image 68 related to acetaldehyde is shown.

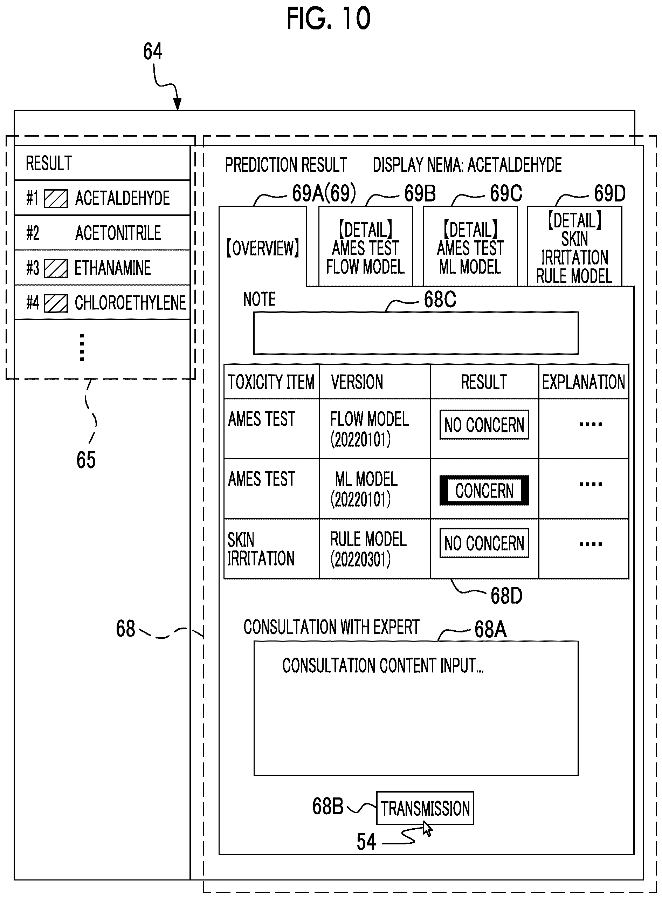

As shown in FIG. 10 as an example, in the entire image 64, the result list image 66 transitions to the result overview image 68. In the example shown in FIG. 10, the result overview image 68 related to acetaldehyde is shown. The result overview image 68 includes a note input region 68C. The note input region 68C is a region in which the user 18 can input any text (for example, precautions regarding a new substance). The content input in the note input region 68C is reflected in a cell of the column "note" of the result list table 66F (see FIG. 9) of the result list image 66.

In addition, a result overview table 68D is shown in the result overview image 68. The result overview table 68D is a table showing the evaluation result for each toxicity item for the selected compound. In the example shown in FIG. 10, items in the columns of the result overview table 68D are "toxicity item", "version", "result", and "explanation" in order from the left. In each row of the result overview table 68D, contents corresponding to the items of the columns are described. In the example shown in FIG. 10, results of three types of toxicity items, namely, the AMES test (flow model and machine learning (ML) model) and skin irritation (rule model), are shown. Here, evaluation results for the toxicity of acetaldehyde show that there is no concern for the AMES test (flow model) and skin irritation (rule model), while there is concern for the AMES test (ML model).

The result overview image 68 includes the input region 68A and the transmission key 68B. The user 18 inputs the inquiry content regarding the evaluation result to the input region 68A. Here, the input region 68A is a text input region (for example, an email form) in which a text indicating the inquiry content can be input. Then, by operating the transmission key 68B, an inquiry notification including the inquiry content is transmitted to a destination (for example, a client terminal of an expert) designated in advance. The inquiry content may include a cause of the evaluation result displayed in the result overview image 68, or a cause of a difference in evaluation results for each toxicity item (for example, a difference in evaluation results of the AMES test (flow model and ML model).

In addition, a tab 69 is displayed in an upper part of the result overview image 68. In a case where the tab 69 is selected, the result overview image 68 and the result detail image 70 related to each toxicity item can be switched. In the example shown in FIG. 10, tabs 69A to 69D are displayed. The tab 69A corresponds to the result overview image 68, the tab 69B corresponds to the result detail image of the AMES test flow model, the tab 69C corresponds to the result detail image of the AMES test ML model, and the tab 69D corresponds to the result detail image of the skin irritation rule model.

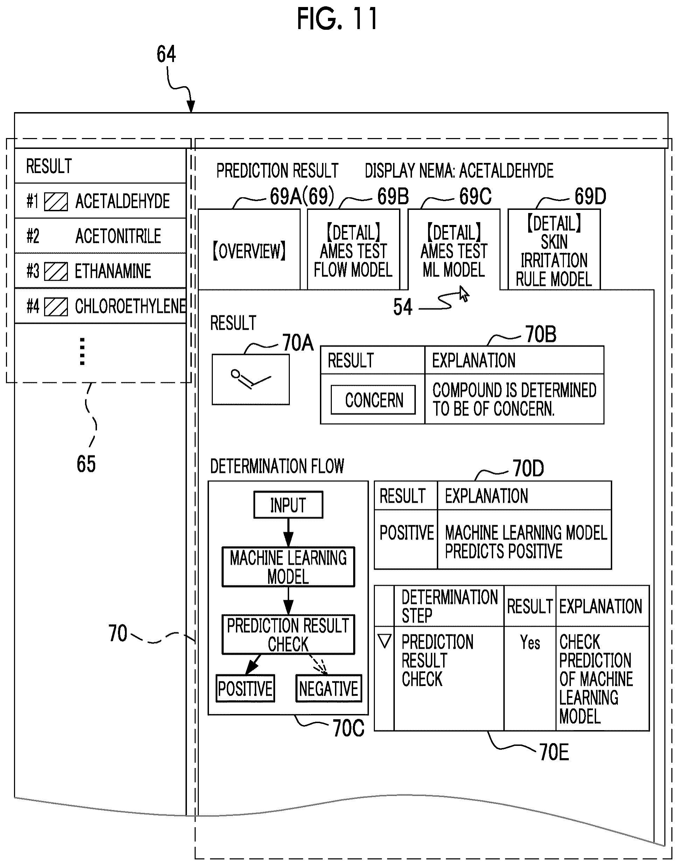

As shown in FIG. 11 as an example, the result overview image 68 transitions to the result detail image 70 in the entire image 64 by selecting the tab 69 corresponding to the result detail image. In the example shown in FIG. 11, by selecting the tab 69C via the pointer 54, the result detail image 70 related to a result of the toxicity evaluation of acetaldehyde using the AMES test ML model is displayed.

The result detail image 70 includes a structural formula image 70A showing a structural formula of the compound and a final result image 70B showing a final result of the toxicity evaluation. In the example shown in FIG. 11, a structural formula of acetaldehyde is shown in the structural formula image 70A, and "concern" as a result and "compound is determined to be of concern" as an explanation are described in the final result image 70B.

In addition, the result detail image 70 includes a flow image 70C showing the toxicity determination flow 33 used for the toxicity evaluation of the compound, a result explanation image 70D for explaining the evaluation result in the toxicity determination flow 33, and a determination step explanation image 70E for explaining the determination content for each determination step included in the toxicity determination flow 33.

In the flow image 70C, a path (that is, an evaluation path) leading to the evaluation result is displayed. In the example shown in FIG. 11, an evaluation path (here, a path leading to "positive") of acetaldehyde in the AMES test ML model is displayed. In addition, in the flow image 70C, the evaluation path is shown to be distinguishable from other paths in the toxicity determination flow 33. In the example shown in FIG. 11, the evaluation path is indicated by a thick line, and the other paths are indicated by a broken line.

In the example shown in FIG. 11, in the determination step explanation image 70E, "prediction result check" is shown as the determination step, and an explanation of the determination content of "check prediction of machine learning model" is shown as the corresponding explanation. In addition, in the result explanation image 70D, "positive" is shown as the result of the toxicity evaluation, and an explanation of the evaluation result that "machine learning model predicts positive" is shown.

In this way, the user 18 can understand a list of toxicity evaluation results of a plurality of compounds and an overview and details of the toxicity evaluation of the selected compound by visually recognizing the result list image 66, the result overview image 68, and the result detail image 70. That is, by checking the evaluation results while transitioning between the result list image 66, the result overview image 68, and the result detail image 70 in the entire image 64, the toxicity evaluation of the compound group can be checked from an overall perspective to a detailed perspective.

Incidentally, in a toxicity evaluation of a compound, it may be necessary to work to understand a tendency such as what kind of toxicity is likely to occur in what kind of compound (that is, the analysis of the toxicity evaluation result), in addition to simply checking the result of the toxicity evaluation. Findings regarding a relationship between these compounds and toxicity are then used in the subsequent development of new compounds. In this case, it is required to perform the analysis in comparison with not only the evaluation results from simulations but also the experimental results performed in the past.

However, the toxicity evaluation software known in the related art does not have a function of performing analysis in the first place, and the analysis is performed using external analysis software by outputting the toxicity evaluation result. In addition, the past experimental results were also separately input into the analysis software and compared with the evaluation results from simulations. As described above, the workload required for the analysis by the user 18 is increased, making it difficult to easily analyze the toxicity evaluation result.

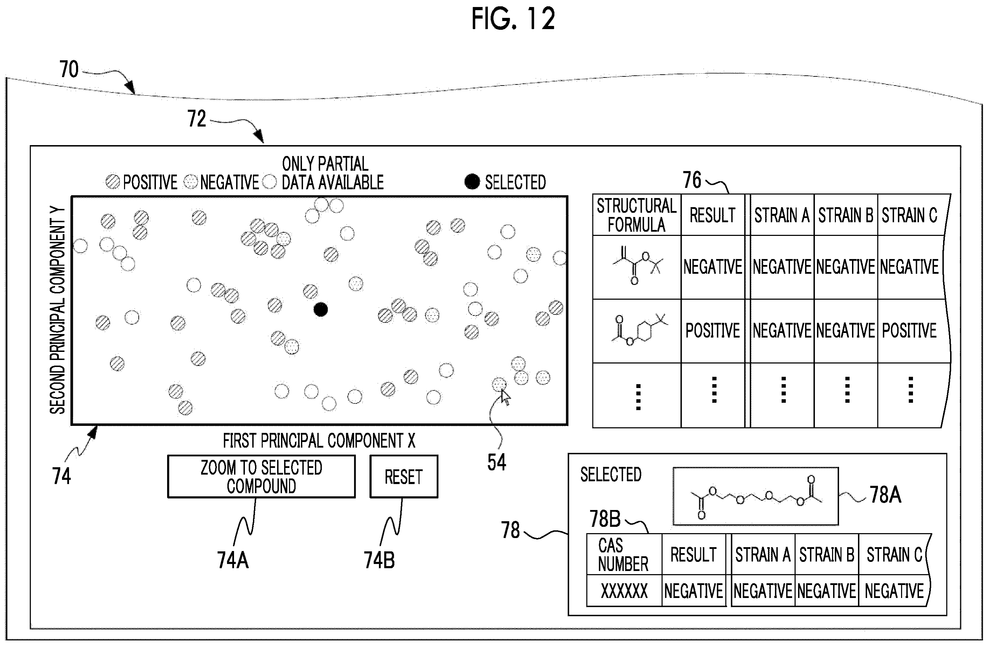

Therefore, in the information processing system 10 according to the present embodiment, as shown in FIG. 12 as an example, the analysis image 72 is displayed to the user 18. The analysis image 72 is displayed in the result detail image 70, which is reached by transitioning from the result list image 66. In the example shown in FIG. 12, in the result overview image 68, the analysis image 72 in a case where the toxicity item of the target compound is the AMES test is shown by selecting the tab 69C of the AMES test.

By visually recognizing the chemical space image 74 included in the analysis image 72, the user 18 can understand the position of the target compound (here, acetaldehyde) in the chemical space and the positional relationship of the other compounds (for example, the reference compound or the compound included in the compound list 58). In addition, by visually recognizing the neighboring compound list image 76, the user 18 can visually recognize the reference compounds present near the target compound in the chemical space in ascending order of a distance to the target compound. This makes it possible to perform analysis by comparing the past experimental results with the evaluation results of the target compound.

In the example shown in FIG. 12, the mark displayed in the chemical space image 74 is selected via the pointer 54. In this case, the compound explanation image 78 is displayed on the analysis image 72. The user 18 can check the details of the toxicity evaluation result of the compound corresponding to the selected mark by visually recognizing the compound explanation image 78. This allows the user 18 to understand the toxicity evaluation results for compounds that he or she is interested in the chemical space.

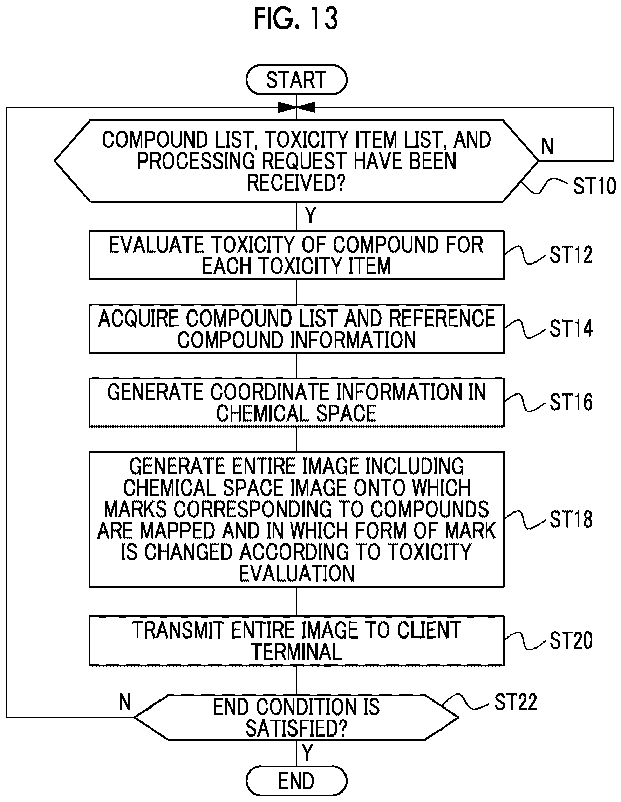

Next, control processing of the server 14 according to the present embodiment will be described with reference to FIG. 13. The server control processing is processing executed by the server 14, and includes the above-described toxicity evaluation processing, coordinate derivation processing, and image generation processing. FIG. 13 is a flowchart showing an example of the server control processing. The flow of the processing shown in FIG. 13 is an example of an "information processing method" according to the technology of the present disclosure.

In the server control processing shown in FIG. 13 as an example, first, in step ST10, the first acquisition unit 30A determines whether or not the compound list 58, the toxicity item list 60, and the processing request have been acquired. In a case where it is determined in the determination in step ST10 that the compound list 58, the toxicity item list 60, and the processing request have been acquired, the determination is affirmative, and the server control processing proceeds to step ST12. In a case where it is determined in the determination in step ST10 that the compound list 58, the toxicity item list 60, and the processing request have not been acquired, the determination is negative, and the server control processing returns to step ST10.

In step ST12, the toxicity evaluation unit 30B performs the toxicity evaluation on each of the compounds indicated by the compound list 58 based on the toxicity item list 60 acquired in step ST10. Specifically, the toxicity evaluation unit 30B inputs the compound information 58A to the toxicity determination flow 33 according to the toxicity item indicated by the toxicity item information 60A. The toxicity determination flow 33 outputs the toxicity evaluation information 62 for each compound. The toxicity evaluation unit 30B acquires the toxicity evaluation information 62. After the process of step ST12 is executed, the server control processing proceeds to step ST14.

In step ST14, the second acquisition unit 30C acquires the compound list 58 and the reference compound information 32E. After the process of step ST14 is executed, the server control processing proceeds to step ST16.

In step ST16, the coordinate setting unit 30D2 generates coordinate information 61 based on the compound list 58 and the reference compound information 32E acquired in step ST14. For example, the coordinate information 61 is generated by performing principal component analysis on the feature amount of each compound. After the process of step ST16 is executed, the server control processing proceeds to step ST18.

In step ST18, the image generation unit 30F generates an entire image 64 based on the coordinate information 61 and the toxicity item list 60. The entire image 64 includes a chemical space image 74. Marks corresponding to compounds are mapped onto the chemical space image 74, and a display form of each mark is changed according to the toxicity evaluation. After the process of step ST18 is executed, the server control processing proceeds to step ST20.

In step ST20, the output unit 30G executes control of outputting the entire image 64 generated by the image generation unit 30F in step ST18 to the client terminal 12. Specifically, the output unit 30E transmits the entire image 64 to the client terminal 12. After the process of step ST18 is executed, the server control processing proceeds to step ST22.

In step ST22, the output unit 30G determines whether or not a condition (hereinafter, referred to as an "end condition") for ending the server control processing is satisfied. Examples of the end condition include a condition in which an instruction to end the server control processing is received. In step ST22, in a case where the end condition is not satisfied, the determination is negative, and the server control processing proceeds to step ST10. In step ST22, in a case where the end condition is satisfied, the determination is affirmative, and the server control processing ends.

As described above, in the information processing system 10 according to the present embodiment, the toxicity evaluation of the compound is performed by the toxicity evaluation unit 30B in the processor 30 of the server 14. In addition, the coordinate setting unit 30D2 sets the position coordinates of the compound in the chemical space. Then, the image generation unit 30F generates an analysis image 72. The analysis image 72 includes a chemical space image 74, and the chemical space image 74 is an image in which marks indicating compounds are mapped onto the chemical space. In addition, the marks are displayed such that the result of the toxicity evaluation for each of the compounds is distinguishable. This makes it easier to understand the relationship between the toxicity evaluation results and the position of the compound in the chemical space for the compound to be analyzed and the reference compound. As a result, this configuration makes it possible to support the user in evaluating the toxicity of the compound.

The toxicity prediction software known in the related art is unable to map compounds onto a chemical space and further unable to display the toxicity evaluation results of the compounds in marks on the chemical space in a distinguishable manner, making it difficult to discriminate the relationship between the positions of the compound to be analyzed and the reference compound in the chemical space and the toxicity evaluation. This configuration makes it easier to visually recognize the relationship between the position of the compound to be analyzed in the chemical space and the toxicity evaluation, thereby supporting matching with the past experimental data and making analysis more efficient.

In addition, in the information processing system 10 according to the present embodiment, the feature amounts used as the coordinate axes in the chemical space are obtained by performing the dimensionality reduction processing on the feature amounts indicating the chemical structure of the compound portion. In the chemical space, the number of the coordinate axes can be reduced by using the feature amounts obtained by the dimensionality reduction processing as the coordinate axes. As a result, the arrangement of the compounds in the chemical space is easily visually recognized.

In addition, in the information processing system 10 according to the present embodiment, in the entire image 64, a neighboring compound list image 76 for displaying details of the toxicity evaluation result for the reference compound is shown separately from the chemical space image 74. In the neighboring compound list image 76, the details of the toxicity evaluation results of the reference compounds are displayed in ascending order of a distance from the compound to be analyzed in the chemical space. The short distance in the chemical space means that there is a commonality in the structural characteristics of the compound and the reference compound. The toxicity evaluation results of the reference compounds having commonality in structural characteristics are displayed, making it easier to consider the relationship between the toxicity and the structure of the target compound.

In addition, in the information processing system 10 according to the present embodiment, in the chemical space image 74,a display range of the chemical space can be changed to a predetermined range centered on a mark indicating the compound to be analyzed by operating the zoom key 74A. It becomes easier to focus on the reference compound present around the compound to be analyzed in the chemical space.

In addition, in the information processing system 10 according to the present embodiment, in a case where a mark corresponding to the reference compound in the chemical space is selected in the chemical space image 74, the compound explanation image 78 showing the result of the toxicity evaluation of the selected reference compound is displayed. This makes it easy for the user 18 to check the evaluation results for the reference compounds that he or she is interested.

In addition, in the information processing system 10 according to the present embodiment, in the chemical space image 74, the result of the toxicity evaluation is made distinguishable by changing the color of the mark in the chemical space according to the result of the toxicity evaluation. This allows the toxicity evaluation results to be distinguished by the difference in color in the chemical space, making it easier to understand the tendency of the toxicity exhibited by the compound.

In addition, in the information processing system 10 according to the present embodiment, in the chemical space image 74, the color of the mark indicating the compound to be analyzed is distinguishable from the color of the mark indicating the reference compound. As a result, since the target compound can be distinguished in the chemical space by the difference in color from the reference compound, the relationship between the position of the target compound in the chemical space and the toxicity evaluation is easily visually recognized.

In addition, in the information processing system 10 according to the present embodiment, the chemical space is a two-dimensional chemical space in which two coordinate axes are set. Since the chemical space is a two-dimensional space, the relationship between the position of the compound to be analyzed in the chemical space and the toxicity evaluation is easily visually recognized. In addition, the load of calculation processing is reduced as compared with a case of drawing a three-dimensional chemical space.

In addition, in the information processing system 10 according to the present embodiment, the chemical space image 74 is displayed by transitioning from the result list image 66 in which results of the toxicity evaluation for a plurality of the compounds are displayed in a list, in a case where at least one compound is selected from the result list image 66. This makes it possible to determine whether or not to perform analysis using the chemical space even in a case where there are a large number of compounds that are candidates for analysis.

In the first embodiment, although an example of a form in which the analysis image 72 is included in the result detail image 70 has been described, the technology of the present disclosure is not limited to this. The analysis image 72 may be displayed as a different image from the result detail image 70.

In addition, the flow of the toxicity evaluation in the toxicity determination flow 33 shown in the first embodiment is merely an example. The flow of the toxicity evaluation in the toxicity determination flow 33 can be appropriately selected or changed by the user. In addition, the determination steps shown in the flow image 70C of the result detail image 70 do not necessarily have to be all of the determination steps included in the toxicity determination flow 33, and may be in a form in which some of the determination steps are displayed. For example, the determination step of interest of the user 18 may be displayed as the flow image 70C.

In addition, in the first embodiment, although an example of a form in which the result overview image 68 and the result detail image 70 related to each toxicity item can be switched by selecting the tab 69 has been described, the technology of the present disclosure is not limited to this. For example, the result overview image 68 may transition to the result detail image 70 related to each toxicity item by clicking the toxicity item of the result overview table 68D shown in the result overview image 68.

In addition, in the first embodiment, although an example of a form in which the transition to the result overview image 68 related to the compound corresponding to the display name is made by selecting the display name of the result list table 66F has been described, the technology of the present disclosure is not limited to this. For example, by selecting the evaluation result of each toxicity item in the result list table 66F, the result list image 66 may transition directly to the result detail image 70 related to each toxicity item without passing through the result overview image 68.

In addition, in the first embodiment, although an example of a form in which the coordinate derivation processing is executed for the compound indicated by the reference compound information 32E has been described, the technology of the present disclosure is not limited to this. For example, in a case where the coordinate information 61 derived in the past is available, it may be used. In addition, the reference compound information 32E may include coordinates of the reference compound in the chemical space.

In addition, in the first embodiment, although an example of a form in which the toxicity evaluation result of the compound corresponding to the mark is made distinguishable by changing the color or pattern of the mark has been described, the technology of the present disclosure is not limited to this. For example, the mark may be made distinguishable by changing the size or shape (for example, a circle, a square, or a triangle) of the mark. In addition, the mark may be made distinguishable by displaying a text indicating the toxicity evaluation result, such as "positive", together with the mark.

In addition, in the first embodiment, although an example of a form in which the mark of the target compound is a black circle and is distinguishable from the reference compound has been described, the technology of the present disclosure is not limited to this. For example, the size and shape of the mark of the target compound may be changed to make it distinguishable, or a text such as "target compound" may be displayed together with the mark to make it distinguishable.

### (First Modification Example)

In the first embodiment, although an example of a form in which the first principal component and the second principal component obtained by the principal component analysis are used as the coordinate axes of the chemical space has been described, the technology of the present disclosure is not limited to this. In the present first modification example, indexes related to a molar mass and lipophilicity of the compound are used as coordinate axes of the chemical space.

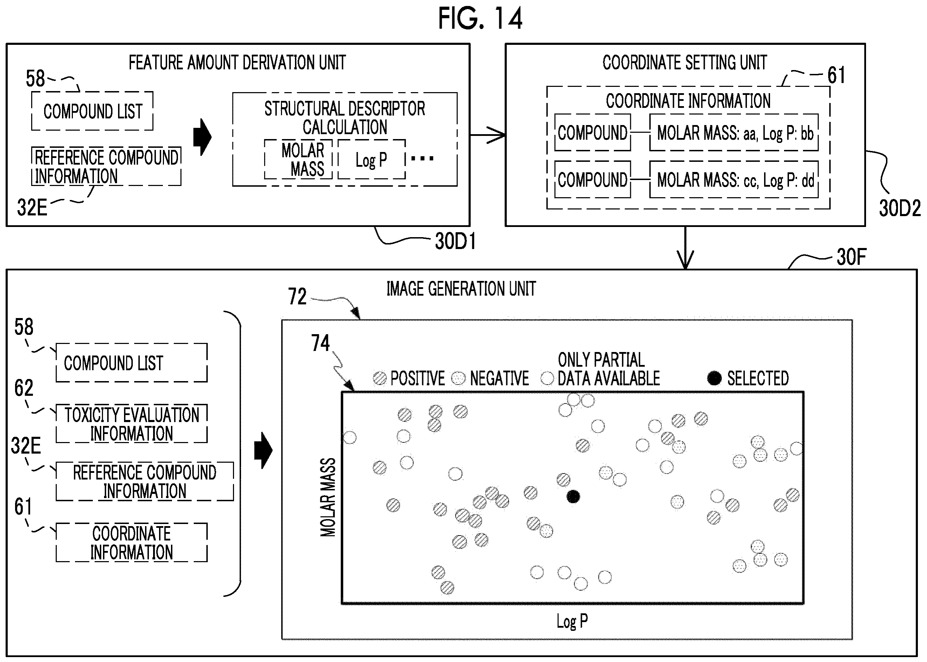

As shown in FIG. 14 as an example, the feature amount derivation unit 30D1 executes the structural descriptor calculation processing on the compound based on the compound list 58 and the reference compound information 32E. The feature amount obtained as a result of the descriptor calculation includes a molar mass and LogP. Here, LogP is an index relating to lipophilicity of a compound. Then, the coordinate setting unit 30D2 generates coordinate information 61 based on the result of the structural descriptor calculation processing. In the example shown in FIG. 14, in the coordinate information 61, the compound is linked to the molar mass and LogP.

The image generation unit 30F generates an analysis image 72 based on the toxicity evaluation information 62, the compound list 58, the reference compound information 32E, and the coordinate information 61. The analysis image 72 includes a chemical space image 74. The image generation unit 30F generates the chemical space image 74 by mapping marks corresponding to the respective compounds onto the chemical space based on the coordinate information 61. In the chemical space indicated by the chemical space image 74, a horizontal axis represents LogP, and a vertical axis represents a molar mass. The user 18 visually recognizes the chemical space image 74 to understand the position of the compound in the chemical space.

As described above, in the information processing system 10 according to the present first modification example, in the chemical space indicated by the chemical space image 74, LogP, which is an index related to lipophilicity, is plotted on the horizontal axis, and the molar mass is plotted on the vertical axis. As a result, in the chemical space defined by the indexes related to the molar mass and the lipophilicity, the relationship between the toxicity evaluation result and the position of the compound in the chemical space is easily understood.

### (Second Modification Example)

In the first embodiment, although an example of a form in which a mark corresponding to a compound is displayed in the chemical space image 74 has been described, the technology of the present disclosure is not limited to this. In the present second modification example, the compounds designated in the chemical space image 74 is filtered out from the compounds displayed in the result list image 66.

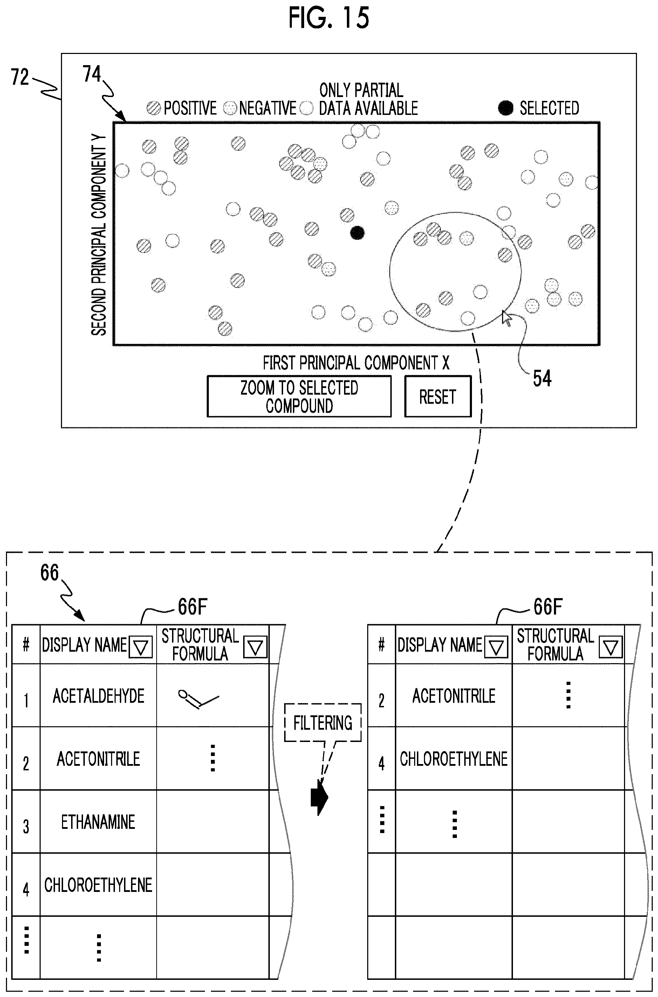

As shown in FIG. 15 as an example, in the chemical space image 74 included in the analysis image 72, the user 18 designates a range via the pointer 54. In the example shown in FIG. 15, in the chemical space image 74, a trajectory along which the pointer 54 is moved forms a circle, thereby designating the range. As a result, compounds corresponding to marks present within the range (that is, inside the circle) are designated.

In a case where the range is designated in the chemical space image 74, the compounds included in the designated range are filtered out in the result list image 66. Specifically, in the chemical space image 74, coordinates of the chemical space corresponding to the designated range are extracted. In the coordinate information 61, the coordinates in the chemical space and the compound are associated with each other. Therefore, the image generation unit 30F filters out the compounds corresponding to the designated range from the compounds being displayed in the result list image 66. Then, the image generation unit 30F updates the result list image 66.

In the example shown in FIG. 15, in the result list table 66F included in the result list image 66, "acetaldehyde, acetonitrile, ethanamine, and chloroethylene" are shown in a state before filtering. Then, in a case where the range is designated in the chemical space image 74, the compounds are filtered out, and, as a result, "acetonitrile and chloroethylene" are shown in the result list table 66F.

As described above, in the information processing system 10 according to the present second modification example, the range is designated by the user 18 in the chemical space displayed in the chemical space image 74. Then, the compounds included in the range selected by the user 18 are filtered out from the compounds that are targets of the toxicity evaluation in the result list table 66F of the result list image 66. This makes it possible to reflect the analysis results of the toxicity using the chemical space (that is, the results of analysis of what kind of toxicity is likely to exhibit in what kind of compound) in the result list image 66.

### [Second Embodiment]

In the first embodiment, although an example of a form in which the toxicity evaluation processing, the coordinate derivation processing, and the image generation processing are performed in the server 14 has been described, the technology of the present disclosure is not limited to this. In the present second embodiment, the toxicity evaluation processing and the coordinate derivation processing are executed in the server 14, and the image generation processing is executed in the client terminal 12. In the present embodiment, the client terminal 12 is an example of an "information processing apparatus" according to the technology of the present disclosure.

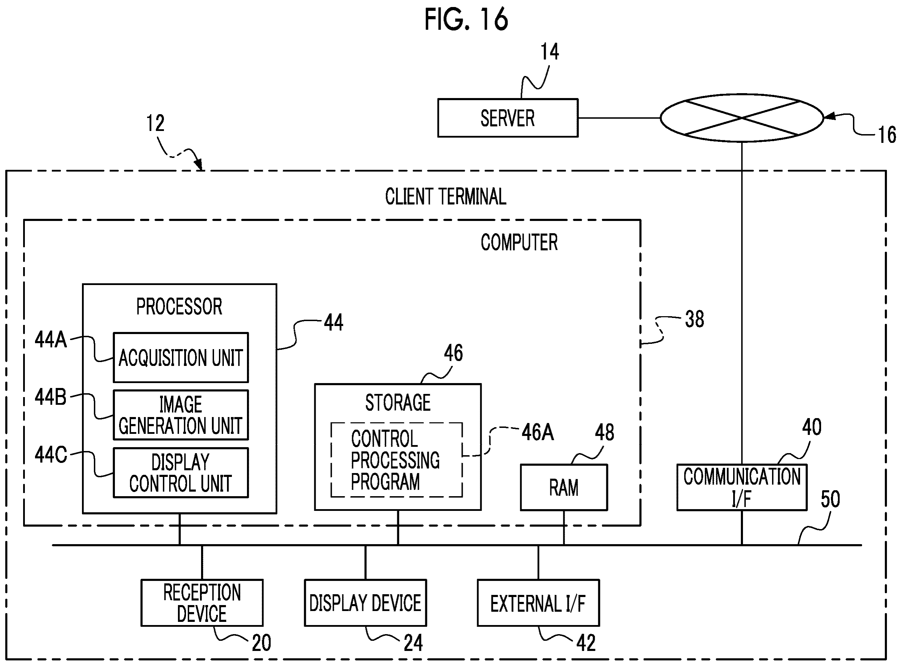

As shown in FIG. 16 as an example, the client terminal 12 comprises a computer 38, the reception device 20, the display device 24, a communication I/F 40, an external I/F 42, and a bus 50. The computer 38 comprises a processor 44, a storage 46, and a RAM 48. The processor 44, the storage 46, the RAM 48, the reception device 20, the display device 24, the communication I/F 40, and the external I/F 42 are connected to the bus 50. In the present embodiment, the processor 44 is an example of a "processor" according to the technology of the present disclosure.

In addition, since a hardware configuration (that is, the processor 44, the storage 46, and the RAM 48) of the computer 38 is basically the same as the hardware configuration of the computer 26, a description of the hardware configuration of the computer 38 will be omitted here.

The communication I/F 40 is connected to the network 16. The communication I/F 40 is responsible for transmitting and receiving information to and from an external communication device (for example, the server 14) via the network 16. For example, the communication I/F 40 transmits information in response to a request from the processor 44 to the external communication device via the network 16. In addition, the communication I/F 40 receives the information transmitted from the external communication device, and outputs the received information to the processor 44 via the bus 50.

The external I/F 42 is responsible for transmitting and receiving various types of information to and from an external device (not shown) present outside the client terminal 12. The external device may be, for example, at least one of a smart device, a personal computer, a server, a universal serial bus (USB) memory, a memory card, or a printer. An example of the external I/F 42 is a USB interface. The external device is connected directly or indirectly to the USB interface.

A control processing program 46A is stored in the storage 46. The control processing program 46A is a program for executing image generation and display control. The processor 30 executes image generation processing by reading out the control processing program 46A from the storage 46 and executing the read-out control processing program 46A on the RAM 48. The image generation processing is realized by the processor 44 operating as an acquisition unit 44A, an image generation unit 44B, and a display control unit 44C. In the present embodiment, the computer 38 is an example of a "computer" according to the technology of the present disclosure, and the control processing program 46A is an example of a "program" according to the technology of the present disclosure.

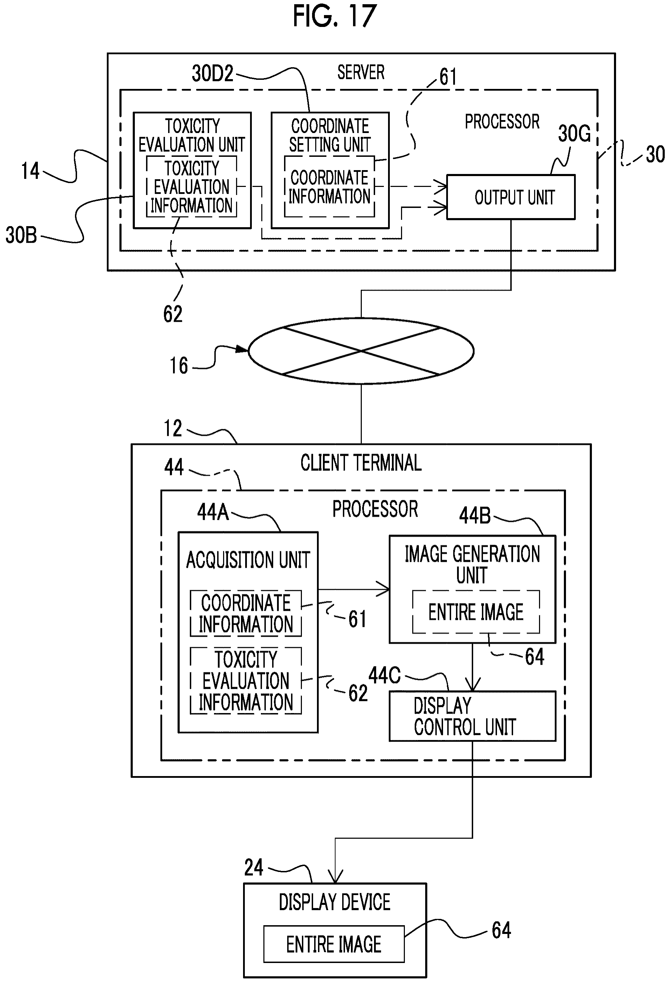

As shown in FIG. 17 as an example, in the processor 30 of the server 14, the toxicity evaluation unit 30B outputs the toxicity evaluation information 62 obtained by the toxicity evaluation processing to the output unit 30E. In addition, the coordinate setting unit 30D2 executes the coordinate setting processing based on the feature amount obtained by the feature amount derivation processing using the feature amount derivation unit 30D1 (see FIG. 6), and outputs the coordinate information 61 obtained by the coordinate setting processing to the output unit 30E. The output unit 30E outputs the toxicity evaluation information 62 and the coordinate information 61 to the client terminal 12 via the network 16.

In the processor 44 of the client terminal 12, the acquisition unit 44A acquires the coordinate information 61 and the toxicity evaluation information 62 via the network 16. Then, the acquisition unit 44A outputs the coordinate information 61 and the toxicity evaluation information 62 to the image generation unit 44B. The image generation unit 44B generates an entire image 64 based on the coordinate information 61 and the toxicity evaluation information 62. Then, the image generation unit 44B outputs the generated entire image 64 to the display control unit 44C. The display control unit 44C performs graphical user interface (GUI) control for displaying the entire image 64, thereby causing the display device 24 to display the entire image 64.

### [Third Embodiment]

In the first embodiment, although an example of a form in which the toxicity evaluation processing, the coordinate derivation processing, and the image generation processing are performed in the server 14 has been described, the technology of the present disclosure is not limited to this. In the present third embodiment, the toxicity evaluation processing, the coordinate derivation processing, and the image generation processing are performed in the client terminal 12. In the present embodiment, the client terminal 12 is an example of an "information processing apparatus" according to the technology of the present disclosure.

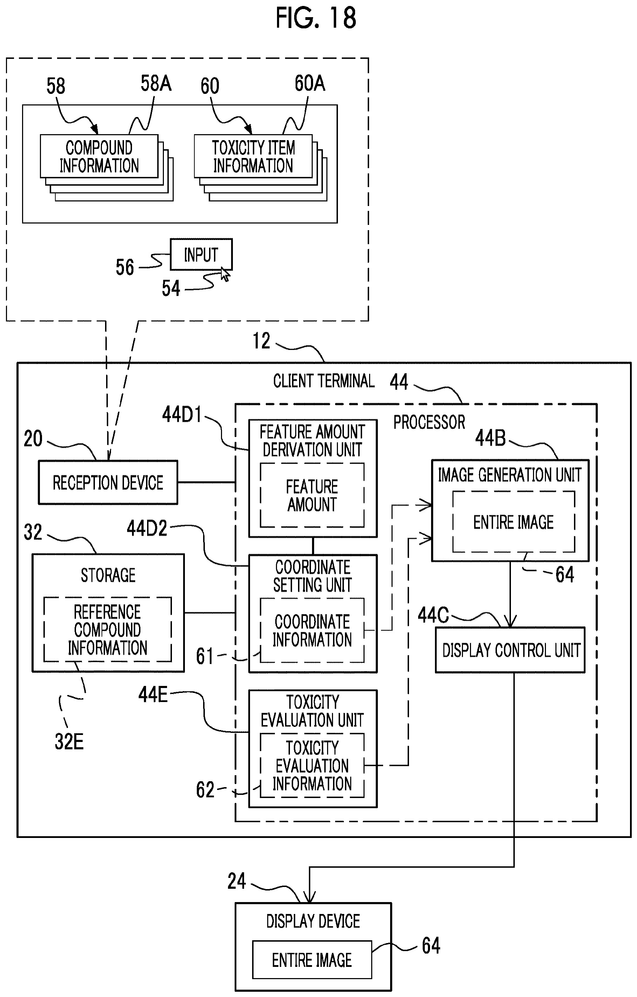

As shown in FIG. 18 as an example, the compound list 58 and the toxicity item list 60 are received in the client terminal 12 via the reception device 20. In the processor 44 of the client terminal 12, the toxicity evaluation unit 44E performs the toxicity evaluation indicated by the toxicity item list 60 on the compound group indicated by the compound list 58. In addition, a feature amount derivation unit 44D1 executes the feature amount derivation processing on the compound indicated by the compound list 58 and the reference compound indicated by the reference compound information 32E. Then, a coordinate setting unit 44D2 generates coordinate information 61 based on the result obtained by the feature amount derivation processing. The image generation unit 44B generates an entire image 64 based on the toxicity evaluation information 62 and the coordinate information 61. The display control unit 44C causes the display device 24 to display the entire image 64.

In the above-described embodiments, although an example of a form in which a two-dimensional chemical space is shown in the chemical space image 74 has been described, the technology of the present disclosure is not limited to this. For example, a three-dimensional chemical space may be shown in the chemical space image 74.

In addition, in the above-described embodiments, although an example of a form in which the toxicity evaluation information 62 is obtained by executing the toxicity evaluation processing has been described, the technology of the present disclosure is not limited to this. For example, the server 14 or the client terminal 12 may receive already obtained toxicity evaluation information 62 (for example, toxicity evaluation information 62 obtained as a result of processing by an external device or toxicity evaluation information 62 obtained in the past), and perform the image generation processing.

In addition, in the above-described embodiments, although an example of a form in which the evaluation processing program 32A, the derivation processing program 32B, and the generation processing program 32C are stored in the storage 32 has been described, the technology of the present disclosure is not limited to this. For example, the evaluation processing program 32A, the derivation processing program 32B, and the generation processing program 32C may be stored in a portable storage medium such as an SSD or a USB memory. The storage medium is a non-transitory computer-readable storage medium. The evaluation processing program 32A, the derivation processing program 32B, and the generation processing program 32C stored in the storage medium are installed in the computer 26. The processor 30 executes control processing of the server 14 in accordance with the evaluation processing program 32A, the derivation processing program 32B, and the generation processing program 32C. Similarly, the control processing program 46A may be stored in a storage medium instead of the storage 46.

In the above-described embodiments, the computers 26 and 38 are exemplified, but the technology of the present disclosure is not limited to this, and a device including an application specific integrated circuit (ASIC), a field programmable gate array (FPGA), and/or a programmable logic device (PLD) may be applied instead of the computers 26 and 38. In addition, instead of the computers 26 and 38, a hardware configuration and a software configuration may be used in combination.

The following various processors can be used as hardware resources for executing the various kinds of processing described in the above-described embodiments. Examples of the processor include a CPU that is a general-purpose processor that functions as a hardware resource for executing the software, that is, the program to execute the toxicity evaluation processing, the coordinate derivation processing, and/or the image generation processing (hereinafter, also simply referred to as "various kinds of processing"). Examples of the processor also include a dedicated electronic circuit that is a processor whose dedicated circuit configuration is specially designed to execute specific processing, such as an FPGA, a PLD, or an ASIC. Any processor includes a memory built therein or connected thereto, and any processor uses the memory to execute various kinds of processing.

The hardware resource for executing the various kinds of processing may be configured by one of the various processors or by a combination of two or more processors of the same type or different types (for example, a combination of a plurality of FPGAs or a combination of a processor and an FPGA). Further, the hardware resource for executing the various kinds of processing may be one processor.

A first example of the configuration in which the hardware resource is configured by one processor is an aspect in which one processor is configured by a combination of one or more processors and software, and this processor functions as the hardware resource for executing the various kinds of processing. Second, as represented by system-on-a-chip (SoC), there is a form in which a processor that realizes the functions of the entire system including a plurality of hardware resources for executing the various kinds of processing with a single integrated circuit (IC) chip. As described above, the various kinds of processing are implemented by using one or more of the various processors as the hardware resource.

Further, specifically, an electronic circuit in which circuit elements, such as semiconductor elements, are combined can be used as the hardware structure of these various processors. The various kinds of processing are merely examples. Accordingly, it is possible to delete an unnecessary step, add a new step, or change a processing order without departing from the gist of the present disclosure.

The content of the above description and the content of the drawings are detailed explanations of the parts relating to the technology of the present disclosure, and are merely examples of the technology of the present disclosure. For example, description related to the above configurations, functions, actions, and effects is description related to an example of configurations, functions, actions, and effects of the parts relating to the technology of the present disclosure. Thus, it is needless to say that unnecessary portions may be deleted, new elements may be added, or replacement may be made to the content of the above description and the content of the drawings without departing from the gist of the technology of the present disclosure. In order to avoid complication and easily understand the parts relating to the technology of the present disclosure, in the content of the above description and the content of the drawings, the description regarding common general technical knowledge which is not necessarily particularly described in terms of embodying the technology of the present disclosure is omitted.

In the present specification, "A and/or B" is synonymous with "at least one of A or B". That is, "A and/or B" may refer to A alone, B alone, or a combination of A and B. In addition, in the present specification, in a case in which three or more matters are expressed with the connection of "and/or", the same concept as "A and/or B" is applied.

All documents, patent applications, and technical standards mentioned in the present specification are incorporated herein by reference to the same extent as in a case in which each document, each patent application, and each technical standard are specifically and individually described by being incorporated by reference.

The disclosure of JP2023-059425 filed on March 31, 2023 is incorporated herein by reference in its entirety.

In regard to the embodiments described above, the following appendices will be further disclosed.

### <Appendix 1>

An information processing apparatus comprising: a processor, in which the processor is configured to generate a display image in which marks indicating a compound group including a target compound, which is a compound to be subjected to an toxicity evaluation, and a plurality of reference compounds, which are compounds to be compared with the target compound in the toxicity evaluation, are mapped onto a chemical space in which a plurality of feature amounts related to a molecular structure of a compound are set as coordinate axes, in which the marks are displayed such that a result of the toxicity evaluation for each of the compounds is distinguishable, and execute control of outputting the generated display image.

### <Appendix 2>

The information processing apparatus according to Appendix 1, in which the plurality of feature amounts are feature amounts obtained by performing dimensionality reduction processing on the compound group.

### <Appendix 3>

The information processing apparatus according to Appendix 1, in which the plurality of feature amounts are indexes related to a molar mass and lipophilicity of the compound.

### <Appendix 4>

The information processing apparatus according to any one of Appendices 1 to 3, in which, in the display image, a display region for displaying details of results of the toxicity evaluation for the reference compounds is provided separately from the chemical space, and in the display region, the details of the results of the toxicity evaluation for the reference compounds are displayed in ascending order of a distance from the target compound in the chemical space.

### <Appendix 5>

The information processing apparatus according to any one of Appendices 1 to 4, in which, in the display image, a display range of the chemical space is changeable to a predetermined range centered on the mark indicating the target compound.

### <Appendix 6>

The information processing apparatus according to any one of Appendices 1 to 5, in which, in the display image, in a case where the mark corresponding to the reference compound in the chemical space is selected, details of a result of the toxicity evaluation for the selected reference compound are displayed.

### <Appendix 7>

The information processing apparatus according to any one of Appendices 1 to 6, in which a display aspect of the mark in the chemical space is changed according to a result of the toxicity evaluation, so that the result of the toxicity evaluation is distinguishable in the display image.

### <Appendix 8>

The information processing apparatus according to any one of Appendices 1 to 7, in which a display aspect of the mark indicating the target compound in the chemical space is distinguishable from a display aspect of the mark indicating the reference compound.

### <Appendix 9>

The information processing apparatus according to any one of Appendices 1 to 8, in which the chemical space is a two-dimensional chemical space in which two coordinate axes are set.

### <Appendix 10>

The information processing apparatus according to any one of Appendices 1 to 9, in which the display image is displayed by transitioning from a list image in which results of the toxicity evaluation for a plurality of the compounds are displayed in a list, in a case where at least one target compound is selected from the list image.

### <Appendix 11>

The information processing apparatus according to Appendix 10, in which, in a case where a user designates a range in the chemical space in the display image, the compounds included in the range are filtered out from the plurality of compounds in the list image.

## Claims

1. An information processing apparatus comprising:
a processor,
wherein the processor is configured to
generate a display image in which marks indicating a compound group including a target compound, which is a compound to be subjected to an toxicity evaluation, and a plurality of reference compounds, which are compounds to be compared with the target compound, are mapped onto a chemical space in which a plurality of feature amounts related to a molecular structure of a compound are set as coordinate axes, in which the marks are displayed such that a result of the toxicity evaluation for each of the compounds is distinguishable, and
execute control of outputting the generated display image.

2. The information processing apparatus according to claim 1,
wherein the plurality of feature amounts are feature amounts obtained by performing dimensionality reduction processing on the compound group.

3. The information processing apparatus according to claim 1,
wherein the plurality of feature amounts are indexes related to a molar mass and lipophilicity of the compound.

4. The information processing apparatus according to claim 1,
wherein, in the display image, a display region for displaying details of results of the toxicity evaluation for the reference compounds is provided separately from the chemical space, and
in the display region, the details of the results of the toxicity evaluation for the reference compounds are displayed in ascending order of a distance from the target compound in the chemical space.

5. The information processing apparatus according to claim 1,
wherein, in the display image, a display range of the chemical space is changeable to a predetermined range centered on the mark indicating the target compound.

6. The information processing apparatus according to claim 1,
wherein, in the display image, in a case where the mark corresponding to the reference compound in the chemical space is selected, details of a result of the toxicity evaluation for the selected reference compound are displayed.

7. The information processing apparatus according to claim 1,
wherein a display aspect of the mark in the chemical space is changed according to a result of the toxicity evaluation, so that the result of the toxicity evaluation is distinguishable in the display image.

8. The information processing apparatus according to claim 1,
wherein a display aspect of the mark indicating the target compound in the chemical space is distinguishable from a display aspect of the mark indicating the reference compound.

9. The information processing apparatus according to claim 1,
wherein the chemical space is a two-dimensional chemical space in which two coordinate axes are set.

10. The information processing apparatus according to claim 1,
wherein the display image is displayed by transitioning from a list image in which results of the toxicity evaluation for a plurality of the compounds are displayed in a list, in a case where at least one target compound is selected from the list image.

11. The information processing apparatus according to claim 10,
wherein, in a case where a user designates a range in the chemical space in the display image, the compounds included in the range are filtered out from the plurality of compounds in the list image.

12. An information processing method comprising:
generating a display image in which marks indicating a compound group including a target compound, which is a compound to be subjected to an toxicity evaluation, and a plurality of reference compounds, which are compounds to be compared with the target compound, are mapped onto a chemical space in which a plurality of feature amounts related to a molecular structure of a compound are set as coordinate axes, in which the marks are displayed such that a result of the toxicity evaluation for each of the compounds is distinguishable; and
executing control of outputting the generated display image.

13. A program for causing a computer to execute a process comprising:
generating a display image in which marks indicating a compound group including a target compound, which is a compound to be subjected to an toxicity evaluation, and a plurality of reference compounds, which are compounds to be compared with the target compound, are mapped onto a chemical space in which a plurality of feature amounts related to a molecular structure of a compound are set as coordinate axes, in which the marks are displayed such that a result of the toxicity evaluation for each of the compounds is distinguishable; and
executing control of outputting the generated display image.
